# EUROPEAN PATENT APPLICATION

(11) **EP 2 985 295 A1**
(43) Date of publication of application: **17.02.2016**
(21) Application number: 14180759.4
(22) Date of filing: 13.08.2014
(51) Int. Cl.: C07K 16/40, A61K 39/395, G01N 33/53

(54) **Antibodies specific for MMP9**

(71) Applicant: Calypso Biotech SA, 1228 Plan-les-Ouates (CH); Merck Patent GmbH, 64293 Darmstadt (DE)
(72) Inventor: Chvatchko Missotten, Yolande, 1232 Confignon (CH); Goffin, Laurence, 01210 Ornex (FR); Leger, Olivier, 74800 Saint Sixt (FR); Dunn, Steven M., 01630 Sergy (FR); Power, Christine, 01710 Thoiry (FR); Maundrell, Kinsey, 1224 Chêne Bougeries (CH)
(74) Representative: reuteler & cie SA

(57) **Abstract**

The present invention relates to new proteins that bind to MMP9 and comprise at least one fragment of a heavy chain variable region and/or at least one fragment of a light chain variable region of an antibody. In particular, the MMP9 binding proteins according to the invention are able to neutralize MMP9 activity and are useful in the prevention and/or treatment of inflammatory and/or autoimmune diseases or cancers.

SEQUENCE LISTING

<110>  CALYPSO BIOTECH SA
       MERCK PATENT GMBH

<120>  ANTIBODIES SPECIFIC FOR MMP9

<130>  P1714EP01

<160>  26

<170>  PatentIn version 3.5

<210>  1
<211>  707
<212>  PRT
<213>  Homo sapiens

<400>  1

<210>  2
<211>  5
<212>  PRT
<213>  Artificial Sequence

<220>
<223>  G03-H-CDR1

<400>  2

Ser Tyr Gly Ile Ser
1               5

<210>  3
<211>  17
<212>  PRT
<213>  Artificial Sequence

<220>
<223>  G03-H-CDR2

<400>  3

Trp Ile Ser Ala Tyr Asn Gly Asn Thr Asn Tyr Ala Gln Lys Leu Gln
1               5                   10                  15

Gly

<210>  4
<211>  7
<212>  PRT
<213>  Artificial Sequence

<220>
<223>  G03-H-CDR3

<400>  4

Asp Tyr Tyr Gly Met Asp Val
1               5

<210>  5
<211>  17
<212>  PRT
<213>  Artificial Sequence

<220>

<223>  G03-H-CDR2-V1

<400>  5

Tyr Ile Ser Ala Tyr Asn Gly Asn Thr Asn Tyr Ala Gln Lys Leu Gln
1               5                   10                  15

Gly

<210>  6
<211>  17
<212>  PRT
<213>  Artificial Sequence

<220>
<223>  G03-H-CDR2-V9

<400>  6

Trp Ile Ser Ala Tyr Asn Ala Asn Thr Asn Tyr Ala Gln Lys Leu Gln
1               5                   10                  15

Gly

<210>  7
<211>  17
<212>  PRT
<213>  Artificial Sequence

<220>
<223>  G03-H-CDR2-V1-V9

<400>  7

Tyr Ile Ser Ala Tyr Asn Ala Asn Thr Asn Tyr Ala Gln Lys Leu Gln
1               5                   10                  15

Gly

<210>  8
<211>  116
<212>  PRT
<213>  Artificial Sequence

<220>
<223>  G03-VH

<400>  8

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Ser Tyr

20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Asn Thr Ala His
65                  70                  75                  80

Met Glu Leu Arg Ser Leu Arg Ser Asp Asp Thr Ala Val Tyr Tyr Cys
                85                  90                  95

Ala Arg Asp Tyr Tyr Gly Met Asp Val Trp Gly Gln Gly Thr Thr Val
            100                 105                 110

Thr Val Ser Ser
        115

<210>  9
<211>  116
<212>  PRT
<213>  Artificial Sequence

<220>
<223>  G03-VH-GL

<400>  9

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Tyr Tyr Gly Met Asp Val Trp Gly Gln Gly Thr Thr Val
            100                 105                 110

Thr Val Ser Ser
        115

<210>  10
<211>  116
<212>  PRT
<213>  Artificial Sequence

<220>
<223>  G03-VH-GL-V1

<400>  10

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Tyr Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Tyr Tyr Gly Met Asp Val Trp Gly Gln Gly Thr Thr Val
            100                 105                 110

Thr Val Ser Ser
        115

<210>  11
<211>  116
<212>  PRT
<213>  Artificial Sequence

<220>
<223>  G03-VH-GL-V9

<400>  11

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr

20                  25                  30

Gly Ile Ser Trp Val Arg Gln Ala Pro Gly Gln Gly Leu Glu Trp Met
        35                  40                  45

Gly Trp Ile Ser Ala Tyr Asn Ala Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Tyr Tyr Gly Met Asp Val Trp Gly Gln Gly Thr Thr Val
            100                 105                 110

Thr Val Ser Ser
        115

<210>  12
<211>  116
<212>  PRT
<213>  Artificial Sequence

<220>
<223>  G03-VH-GL-V1-V9

<400>  12

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Tyr Ile Ser Ala Tyr Asn Ala Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Tyr Tyr Gly Met Asp Val Trp Gly Gln Gly Thr Thr Val
            100                 105                 110

Thr Val Ser Ser
        115

<210>  13
<211>  13
<212>  PRT
<213>  Artificial Sequence

<220>
<223>  G06-L-CDR1

<400>  13

Ser Gly Ser Thr Phe Asn Ile Gly Ser Asn Thr Val Asn
1               5                   10

<210>  14
<211>  7
<212>  PRT
<213>  Artificial Sequence

<220>
<223>  G06-L-CDR2

<400>  14

Ser Asp Ser His Arg Pro Ser
1               5

<210>  15
<211>  9
<212>  PRT
<213>  Artificial Sequence

<220>
<223>  G06-L-CDR3

<400>  15

Gln Ala Trp Asp Ser Ser Ala Val Val
1               5

<210>  16
<211>  109
<212>  PRT
<213>  Artificial Sequence

<220>
<223>  G06-VL

<400>  16

Gln Ala Val Leu Thr Gln Pro Pro Ser Thr Ser Gly Thr Pro Gly Gln
1               5                   10                  15

Arg Val Thr Ile Ser Cys Ser Gly Ser Thr Phe Asn Ile Gly Ser Asn

20                  25                  30

Thr Val Asn Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Asn Ser Trp Asn Thr Ala Thr Leu Thr Ile Ser Arg Val Glu
65                  70                  75                  80

Val Gly Asp Glu Gly Asp Tyr Tyr Cys Gln Ala Trp Asp Ser Ser Ala
                85                  90                  95

Val Val Phe Gly Gly Gly Thr Lys Val Thr Val Leu Gly
            100                 105

<210>  17
<211>  109
<212>  PRT
<213>  Artificial Sequence

<220>
<223>  G06-VL-GL1

<400>  17

Gln Ala Val Leu Thr Gln Pro Pro Ser Ala Ser Gly Thr Pro Gly Gln
1               5                   10                  15

Arg Val Thr Ile Ser Cys Ser Gly Ser Thr Phe Asn Ile Gly Ser Asn
            20                  25                  30

Thr Val Asn Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Asn Ser Trp Asn Thr Ala Ser Leu Ala Ile Ser Gly Leu Gln
65                  70                  75                  80

Ser Glu Asp Glu Ala Asp Tyr Tyr Cys Gln Ala Trp Asp Ser Ser Ala
                85                  90                  95

Val Val Phe Gly Gly Gly Thr Lys Val Thr Val Leu Gly
            100                 105

<210>  18
<211>  15

<212>  DNA
<213>  Artificial Sequence

<220>
<223>  G03-H-CDR1

<400>  18
agttacggca tctct                                                        15


<210>  19
<211>  51
<212>  DNA
<213>  Artificial Sequence

<220>
<223>  G03-H-CDR2

<400>  19
tggatctccg cctataatgg taatacaaac tatgcgcaga aactccaagg c                51


<210>  20
<211>  21
<212>  DNA
<213>  Artificial Sequence

<220>
<223>  G03-H-CDR3

<400>  20
gactattacg gcatggacgt g                                                 21


<210>  21
<211>  51
<212>  DNA
<213>  Artificial Sequence

<220>
<223>  G03-H-CDR2-V1

<400>  21
tatattagcg cttacaacgg aaacaccaat tacgcccaga agctccaggg c                51


<210>  22
<211>  51
<212>  DNA
<213>  Artificial Sequence

<220>
<223>  G03-H-CDR2-V1-V9

<400>  22
tatattagtg catataacgc aaataccaat tacgctcaaa aactccaagg a                51


<210>  23
<211>  39
<212>  DNA
<213>  Artificial Sequence

<220>
<223>  G06-L-CDR1

<400>  23
tcgggttcta catttaatat aggaagtaat actgtgaat                              39


<210>  24
<211>  21
<212>  DNA
<213>  Artificial Sequence

<220>
<223>  G06-L-CDR2

<400>  24
agcgacagtc acagaccatc t                                                 21


<210>  25
<211>  27
<212>  DNA
<213>  Artificial Sequence

<220>
<223>  G06-L-CDR3

<400>  25
caagcctggg acagctcagc tgtcgta                                           27


<210>  26
<211>  326
<212>  PRT
<213>  Artificial Sequence

<220>
<223>  engineered human IgG4 heavy chain constant region

<400>  26

Ala Ser Thr Lys Gly Pro Ser Val Phe Pro Leu Ala Pro Cys Ser Arg
1               5                   10                  15


Ser Thr Ser Glu Ser Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60


Leu Ser Ser Val Val Thr Val Pro Ser Ser Ser Leu Gly Thr Lys Thr
65                  70                  75                  80


Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Lys Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly
                325

## Description

### Field of the Invention

The present invention relates to antibodies and fragments thereof that specifically bind to the multi-domain metalloenzyme MMP9 (gelatinase B) and are, in particular, able to neutralize the activity of said protein, as well as to uses thereof as therapeutics.

### Background of the Invention

The matrix metalloproteinase (MMP) family consists of at least 25 structurally related, soluble or membrane bound zinc-dependent endopeptidases that are broadly involved in the remodelling of the extracellular matrix (ECM) and in the functional regulation of various bioactive molecules.

All MMPs possess a prototype structure that includes a pro-domain that maintains the MMP in an inactive form and a catalytic domain that acts on a broad spectrum of extracellular matrix components.

Matrix metallopeptidase 9 (MMP9), also known as 92 kDa type IV collagenase or gelatinase B (GELB), is a member of the MMP family enzymes responsible for the degradation of denatured and basement membrane collagens.

The general domain structure of MMP9 comprises a secretory leader sequence, an inhibitory pro-domain required for catalytic latency, a 'split' catalytic domain containing three fibronectin type II-like repeat loops that together form a collagen binding domain (CBD), a hyperglycosylated proline-rich linker (also referred to as the OG domain), and a haemopexin-like repeat C-terminal domain (PEX).

Many acute inflammatory and autoimmune disease states, fibrotic conditions and invasive cancer, are associated with the presence of excessive MMP9 (Hu et al, 2007, Nature Reviews Drug Discovery, 6, 480-498 ; Ram et al, 2006, J. Clin. Immunol., (26)4: 299-307 *;* Ai Zheng, 2003, Chinese journal of cancer, 22(2):178-84 *;* Baugh et al, 1999, Gastroenterology, 117: 814-822 *;* Santos et al, 2013, Biochem Biophys Res Commun. Sep 6; 438(4): 760-4*) ;* Herszényi et al, 2012, Int. J. Mol. Sci., 13: 13240-13263 *;* Lijnen, 2001, Thromb Haemost 86: 324-33 *;* Rosell et al., 2005, Stroke 36: 1415-20 *;* Whatling et al., 2004, Arterioscler Thromb Vasc Biol 24: 10-11 *;* Yasmin et al., 2005, Arterioscler Thromb Vasc Biol 25:372-8 *;* Vassiliadis et al., 2011, BMC Dermatol. 11: 6) and, thus, this enzyme has received considerable attention as a prospective target for therapeutic intervention.

Historically, strategies for MMP blockade have focused on the design of small molecule inhibitors that interact intimately with the catalytic site of the activated enzyme. To date, this approach has failed to translate into the expected clinical benefit partly due to dose-limiting toxicity and severe side effects such as musculoskeletal syndrome. As the architecture of the MMP9 catalytic site is highly conserved across the MMP family, this contra-indication may be attributable to a lack of MMP target selectivity at therapeutic doses.

Antibodies or antibody fragments are likely to interact with, and occlude a far larger portion of the MMP9 structure than active-site directed small molecules providing higher target inhibitory selectivity.

Some antibodies specific for MMP9 have been described in the prior art such as mouse AB0041, humanized AB0045, and humanized AB0046 (WO 2013/130078) as well as 539A-M0240-B03 (US 2009/0311245), M0166-F10 (US 2009/0311245 and US 2011/0135573), 539A-M0237-D02 (US 2009/0297449 and US 2011/0135573). Some of the antibodies of the prior art have been described as binding to both MMP9 and MMP2.

Therefore, there remains a need for the development of novel therapeutic agents which show a high affinity and specificity for MMP9 and exhibit a weak or limited affinity and/or specificity to other MMPs such as MMP2, show improved cross-reactivity to non-human MMP9 orthologs, and possess other additional properties such as reduced immunogenicity in humans and/or higher stability, which rend them particularly suitable for therapeutic applications in humans.

### Summary of the Invention

The present invention is directed towards proteins that bind to MMP9, in particular human MMP9 and comprise at least one fragment of a heavy chain variable region and/or at least one fragment of a light chain variable region of an antibody as described herewith.

A first aspect of the invention provides an isolated antibody specific for MMP9 or antigen-binding fragment thereof comprising a heavy chain variable region comprising:
(i) a heavy chain CDR1 of SEQ ID NO: 2 or a variant thereof wherein 1, 2, or 3 amino acids of said heavy chain CDR1 is substituted by a different amino acid;
(ii) a heavy chain CDR2 of SEQ ID NO: 3 or a variant thereof wherein 1, 2, or 3 amino acids of said heavy chain CDR2 is substituted by a different amino acid;
(iii) a heavy chain CDR3 of SEQ ID NO: 4 or a variant thereof wherein 1, 2, or 3 amino acids of said heavy chain CDR3 is substituted by a different amino acid.

A more particular aspect of the invention provides an isolated antibody as described above, further comprising a light chain variable region comprising:
(i) a light chain CDR1 of SEQ ID NO: 13 or a variant thereof wherein 1, 2, or 3 amino acids of said light chain CDR1 is substituted by a different amino acid;
(ii) a light chain CDR2 of SEQ ID NO: 14 or a variant thereof wherein 1, 2, or 3 amino acids of said light chain CDR2 is substituted by a different amino acid;
(iii) a light chain CDR3 of SEQ ID NO: 15 or a variant thereof wherein 1, 2, or 3 amino acids of said light chain CDR3 is substituted by a different amino acid.

A second aspect of the invention relates to an isolated nucleic acid molecule encoding an antibody or antigen binding fragment thereof as described herewith.

A third and fourth aspects of the invention relate to a recombinant expression vector comprising said nucleic acid molecule, and to a host cell comprising said recombinant vector, respectively.

A fifth aspect of the invention relates to a process for producing antibodies or fragments thereof as described herewith comprising culturing a host cell transformed with an expression vector comprising a nucleic sequence that encodes said antibodies or fragments thereof under conditions sufficient to promote expression of said antibodies or fragments thereof.

A sixth aspect of the invention provides a pharmaceutical composition comprising one or more of (i) an antibody specific for MMP9 or antigen-binding fragment thereof, (ii) a nucleic acid, (iii) a vector, and/or (iv) a host cell, as described herewith, and at least one pharmaceutically acceptable carrier.

A seventh aspect of the invention relates to an imaging composition or a diagnosis composition comprising one or more anti-MMP9 antibody or antigen-binding fragment thereof as described herewith.

An eighth aspect of the invention is a kit comprising one or more anti-MMP9 antibody or antigen-binding fragment thereof as described herewith.

A ninth aspect of the invention relates to an antibody or formulation thereof according to the invention for use in the prevention and/or treatment of an inflammatory and/or autoimmune disease or a cancer or tumor.

A tenth aspect relates to a method of preventing and/or treating an inflammatory and/or autoimmune disease or a cancer comprising administering in a subject in need thereof a therapeutically effective amount of said antibody or fragment thereof or said pharmaceutical composition.

Other features and advantages of the invention will be apparent from the following detailed description.

### Description of the figures

**Figure 1** shows a schematic diagram of molecular domain structure of the human MMP9 protein. Numbers indicate amino acid positions on the immature MMP9 protein amino acid sequence.
**Figure 2** shows alignment of amino acid sequences of human MMP9, Cynomologus monkey (cyno) MMP9, rat MMP9 and mouse MMP9. (*) indicates positions which have a single, fully conserved residue, (:) indicates conservation between groups of strongly similar properties - scoring > 0.5 in the Gonnet PAM 250 matrix, (.) indicates conservation between groups of weakly similar properties - scoring ≤ 0.5 in the Gonnet PAM 250 matrix.
**Figure 3** shows sequence alignment of exemplary human heavy chain variable region comprised in the anti-MMP9 antibodies according to the invention: G03-VH, G03-VH-GL, G03-VH-GL-V1, G03-VH-GL-V9, G03-VH-GL-V1-V9. CDRs are underlined.
Annotations are identical to the ones described in Figure 2.
**Figure 4** shows sequence alignment of exemplary human light chain variable region comprised in the anti-MMP9 antibodies according to the invention: G06-VL, G06-VL-GL1. CDRs are underlined. Annotations are identical to the ones described in Figure 2.
**Figure 5** is a graph that depicts neutralizing activity of exemplary anti-MMP9 antibodies according to the invention towards human proMMP9 (A) and human mature MMP9 (B). Antibodies tested: G03-VH-GL/G06-VL-GL1 (•), G03-VH-GL-V1/G06-VL-GL1 (▲), G03-VH-GL-V1-V9/G06-VL-GL1 (∇).
**Figure 6** is a graph that reports selectivity of exemplary anti-MMP9 antibodies according to the invention towards various matrix metalloproteinases.

### Detailed Description of the invention

### Definitions

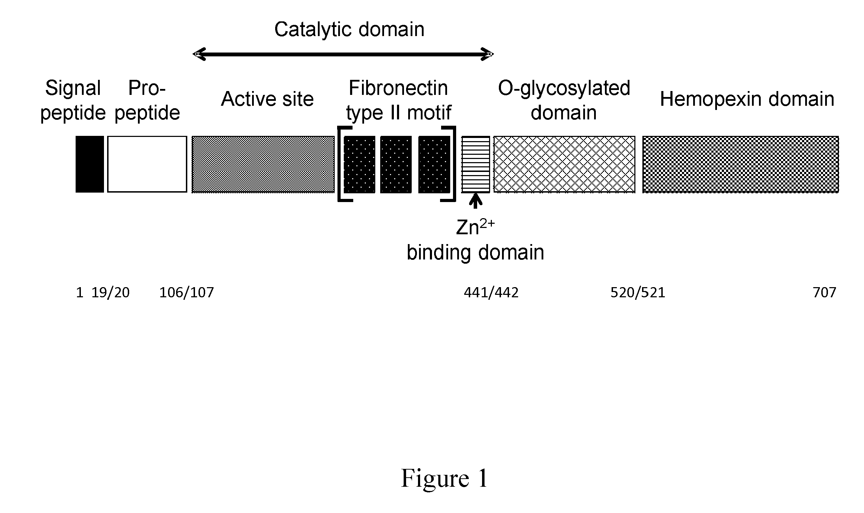

The term "Matrix metalloproteinase 9", abbreviated "MMP9", also known as 92 kDa type IV collagenase, 92 kDa gelatinase or gelatinase B (GELB), is an enzyme that, in humans, is encoded by the *MMP9* gene whose sequence is disclosed under NCBI accession number ENSG00000100985. The immature form of human MMP9 comprises 707 amino acids in total, its amino acid sequence is available under NCBI accession number NP_004985.2 (SEQ ID NO: 1). The general domain structure of MMP9 comprises a secretory leader sequence (residues 1-19 on SEQ ID NO: 1), an inhibitory pro-domain required for catalytic latency (residues 20-106 on SEQ ID NO: 1), a 'split' catalytic domain (residues 107-441 on SEQ ID NO: 1) containing three fibronectin type II-like repeat loops that together form a collagen binding domain (CBD), a hyperglycosylated proline-rich linker (also referred to as the OG domain) (residues 442-520 on SEQ ID NO: 1), and a haemopexin-like repeat C-terminal domain (PEX) (residues 521-707 on SEQ ID NO: 1) (Rowsell et al., 2002, J Mol Biol 319:173-81) (Figure 1). MMP9 is secreted and maintained as an inactive, latent form by a pro-domain. Proteolytic removal of the pro-domain activates the enzymatic activity of MMP9. Its catalytic domain contains a gelatin-binding region, which provides specific affinity for gelatin. In addition to gelatin, MMP9 has various substrates including collagens (e.g. collagen IV, and collagen V), elastin, galectin3, entactin and ICAM-1 *(*Ram et al, 2006, J. Clin. Immunol. 26, 299-307), and cytokines and chemokines *(*Opdenakker et al, Trends Immunol. 2001: 22:527-81).

The term "antibody" as referred to herein designates a polypeptide that binds to an antigen. This includes whole antibodies and any antigen binding fragments. The term "antibody" is used in its broadest sense and includes monoclonal antibodies, polyclonal antibodies, human antibodies, humanized antibodies, chimeric antibodies, and further engineered antibodies as long as the characteristic properties of the invention are retained, in particular the ability of binding to the target antigen, more specifically to the same epitope of MMP9 as the one recognized by the antibodies of the invention. Examples of antibodies and fragments thereof include a variable domain fragment ("Fv", consisting of the VH and VL domains of a single arm of an antibody), Fab fragment (monovalent fragment consisting of the VH, VL, CH1 and CL domains), Fab₂ fragment (bivalent), Fab₃ fragment (trivalent), Fab' fragment (Fab with hinge region), F(ab')₂ fragment (bivalent fragment including two Fab fragments linked by a disulfide bridge at the hinge region), Fd fragment (consisting of the VH and CH1 domains), rIgG (reduced IgG or half-IgG), diabodies, triabodies, tetrabodies, minibodies, domain antibodies (dAb), nanobodies, monovalent antibodies, divalent or multivalent antibodies comprising a fragment of more than one antibody, single chain variable fragment (ScFv), bis-scFv (bispecific), and derivatives of antibodies such as disulfide stabilized Fv fragments, CDR-comprising peptides, as well as epitope-binding fragments of any of the above (Holliger and Hudson, 2005, Nature Biotechnology, 23(9): 1126-1136)*.* An antibody refers to a glycoprotein comprising at least two heavy (H) chains and two light (L) chains inter-connected by disulfide bonds, or an antigen-binding fragment thereof. Each heavy chain comprises a heavy chain variable region (VH) and a heavy chain constant region (CH). Each light chain comprises a light chain variable region (VL) and a light chain constant region (CL). In mammalians, the heavy chain can either be alpha (α), delta (δ), epsilon (ε), gamma (γ) or mu (µ), which defines the class of antibody IgA, IgD, IgE, IgG and IgM, respectively. In mammalians, the light chain can either be lambda (λ) or kappa (κ). In mammalians, depending on the class of antibody, the heavy chain constant region comprises three immunoglobulin domains, CH1, CH2, and CH3 (for IgA, IgD, IgG) or four immunoglobulin domains, CH1, CH2, CH3, and CH4 (for IgE and IgM). The light chain constant region comprises one immunoglobulin domain, CL. An antibody can have the structure of an IgA, IgG, IgE, IgD and IgM as well as any subtype thereof. Antibodies may be from any source including in particular primate (human and non-human primate) and primatized sources.

The term "variable domain" (variable domain of a light chain (VL), variable domain of a heavy chain (VH)) as used herein refers to each of the pair of light and heavy chain domains which are involved directly in binding the antibody to the antigen. The variable light and heavy chain domains have the same general structure and each domain comprises four framework ("FR") regions whose sequences are widely conserved, connected by three "hypervariable regions" called "complementary determining regions" or "CDRs". The framework regions adopt a β-sheet conformation and the CDRs may form loops connecting the β-sheet structure. The CDRs in each chain are held in their three-dimensional structure by the framework regions and form together with the CDRs from the other chain the antigen binding site. The term "antigen-binding portion of an antibody" when used herein refers to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus: the domains FR1, CDR1, FR2, CDR2, FR3, CDR3, and FR4. The residues of the CDR and FR regions are conventionally numbered according to the standard definition of Kabat et al (Sequences of Proteins of Immunological Interest, 5th ed., Public Health Service, National Institutes of Health, Bethesda, MD (1991), Publication No. 91-3242*).* This numbering system is used in the present specification except where otherwise indicated. The Kabat residue designations do not always correspond directly to the linear numbering of the amino acid residues. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35 (CDR- H1), residues 50-65 (CDR-H2) and residues 95-102 (CDR-H3) according to the Kabat numbering system. The CDRs of the light chain variable domain are located at residues 24-34 (CDR-L1), residues 50-56 (CDR-L2) and residues 89-97 (CDR-L3) according to the Kabat numbering system.

In the present application, unless specified otherwise, for all human immunoglobulin heavy and light chain variable domains, numbering is according to the "Kabat numbering system" (Sequences of Proteins of Immunological Interest, 5th ed., Public Health Service, National Institutes of Health, Bethesda, MD (1991), Publication No. 91-3242*).*

In the present application, unless specified otherwise, for all human immunoglobulin heavy chain constant domains, numbering is according to the "EU numbering system" (Edelman et al, 1969, Proc Natl Acad Sci, 63(1): 78-85).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. The modifier "monoclonal" indicates the character of the antibody as being obtained from a substantially homogeneous population of antibodies, and is not to be construed as requiring production of the antibody by any particular method.

The term "chimeric antibody" generally refers to an antibody comprising a variable region from one source or species and at least a portion of a constant region derived from a different source or species, usually prepared by recombinant DNA techniques. A typical example of chimeric antibodies includes those comprising a murine variable region and a human constant region. As defined herewith this term also includes an antibody comprising at least one of the CDRs of a first human antibody and at least a portion of a constant region of a second human antibody. It also includes an antibody comprising heavy chain CDR1, CDR2, and CDR3 of a first human antibody and light chain CDR1, CDR2, and CDR3 of a second human antibody.

The term "humanized antibody" designates antibodies from a non-human species having one or more complementarity determining regions (CDRs) from said non-human species and a framework region from a human immunoglobulin molecule. Humanized antibodies may optionally further comprise one or more framework residues derived from the non-human species from which the CDRs were derived.

The term "human antibody" or "fully human antibody" refers to antibodies in which the variable regions and the constant regions of both the heavy and the light chains are all of human origin, or substantially identical to sequences of human origin, but not necessarily from the same antibody.

The term "isolated antibody" refers to an antibody that has been separated from a component of its natural environment. For instance, an isolated antibody has been purified to greater than 95% or 99% purity as determined by methods in the art (see e.g. Flatman et al, 2007, J Chromatogr B Analyt Technol Biomed Life Sci, 848: 79-87) including electrophoretic (e.g. SDS-PAGE, isoelectric focusing, capillary electrophoresis) or chromatographic (e.g. ion exchange or reverse phase HPLC (high performance liquid chromatography) methods.

The terms "polynucleotide" or "nucleic acid molecule" refers to a polymer comprising nucleotides. Examples of nucleic acid molecules include DNA, RNA, locked nucleic acid (LNA), complementary DNA (cDNA).

"Polypeptide" is understood as a peptide, an oligopeptide, an oligomer or a protein comprising at least two amino acids joined to each other by a normal or modified peptide bond, such as in the cases of the isosteric peptides, for example. A polypeptide can be composed of amino acids other than the 20 amino acids defined by the genetic code. A polypeptide can equally be composed of amino acids modified by natural processes, such as post-translational maturation processes or by chemical processes, which are well known to a person skilled in the art. Such modifications are fully detailed in the literature. These modifications can appear anywhere in the polypeptide: in the peptide skeleton, in the lateral chain or even at the carboxy- or amino- terminal ends. For example, polypeptide modifications is understood to include acetylation, acylation, ADP-ribosylation, amidation, covalent fixation of flavine, covalent fixation of heme, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bond formation, demethylation, cysteine formation, pyroglutamate formation, formylation, gamma-carboxylation, glycosylation including pegylation, GPI anchor formation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature (Proteins Structure and Molecular Properties (1993) 2nd Ed., T. E. Creighton, New York*;* Post-translational Covalent Modifications of Proteins (1983) B. C. Johnson, Ed., Academic Press, New York*;* Seifter et al. (1990) Analysis for protein modifications and nonprotein cofactors, Meth. Enzymol. 182: 626-646 *and* Rattan et al., (1992) Protein Synthesis: Post-translational Modifications and Aging, Ann NY Acad Sci, 663: 48-62)*.*

"Isolated polynucleotide" or "isolated polypeptide" is understood as a polynucleotide or a polypeptide such as previously defined which is isolated from the human body or otherwise produced by a technical process.

The term "variant" can apply to a polynucleotide and/or a polypeptide. For instance, a variant of a peptide or polypeptide, as referred to herein means a peptide or polypeptide substantially homologous to the referenced peptide sequence, but which has an amino acid sequence different from that of the referenced sequence because of one or more amino acid deletions, insertions and/or substitutions. Substantially homologous means a variant amino acid sequence which is identical to the referenced peptide sequence except for the deletion, insertion and/or substitution of a few amino acids, e.g. 1, 2, 3, 4, 5, or 6 amino acids. Substantially homologous means a variant amino acid sequence that is at least 80%, at least 85%, at least 90%, at least 95%, at least 96%, at least 97%, at least 98% or at least 99% identical to the referenced amino acid sequence. A variant nucleic acid sequence can be at least 80%, at least 85%, at least 90%, at least 95%, at least 96%, at least 97%, at least 98% or at least 99% identical to the referenced nucleic acid sequence. The identity of two amino acid sequences or of two nucleic acid sequences can be determined by visual inspection and/or mathematical calculation, or more easily by comparing sequence information using known computer program used for sequence comparison such as Clustal package version 1.83. A variant may comprise a sequence having at least one conservatively substituted amino acid, meaning that a given amino acid residue is replaced by a residue having similar physiochemical characteristics. Generally, substitutions for one or more amino acids present in the original polypeptide should be made conservatively. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known (Kyte, et al, 1982, J. MoI. Biol., 157: 105- 131). For example, a "conservative amino acid substitution" may involve a substitution of a native amino acid residue with a non-native residue such that there is little or no effect on the polarity or charge of the amino acid residue at that position. Desired amino acid substitutions (whether conservative or non-conservative) can be determined by those skilled in the art at the time such substitutions are desired. Exemplary amino acid substitutions are presented in Table 1 below. The term "variant" also includes a peptide or polypeptide substantially homologous to the referenced peptide sequence, but which has an amino acid sequence different from that of the referenced sequence because one or more amino acids have been chemically modified or substituted by amino acids analogs. This term also includes glycosylated polypeptides.

**Table 1**

| **Original residues** | **Examples of substitutions** |
|---|---|
| Ala (A) | Val, Leu, He |
| Arg (R) | Lys, Gln, Asn |
| Asn (N) | Gln |
| Asp (D) | Glu |
| Cys (C) | Ser, Ala |
| Gln (Q) | Asn |
| Glu (E) | Asp |
| Gly (G) | Pro, Ala |
| His (H) | Asn, Gln, Lys, Arg |
| Ile (I) | Leu, Val, Met, Ala, Phe |
| Leu (L) | Ile, Val, Met, Ala, Phe |
| Lys (K) | Arg, Gln, Asn |
| Met (M) | Leu, Ile, Phe |
| Phe (F) | Leu, Val, Ile, Ala, Tyr |
| Pro (P) | Ala, Gly |
| Ser (S) | Thr, Ala, Cys |
| Trp (W) | Phe, Tyr |
| Thr (T) | Ser |
| Tyr (Y) | Trp, Phe, Thr, Ser |
| Val (V) | He, Met, Leu, Phe, Ala |

The term "epitope" includes any polypeptide determinant capable of specific binding to an antibody. In certain embodiments, epitope determinant includes chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

As used herewith the term "bind" or "binding" of an antibody to a target antigen means an at least temporary interaction or association of said antibody with, or to, said target antigen (such as MMP9) or with, or to, fragments of said target antigen comprising an epitope recognized by said antibody.

The terms "selectively binds", "specifically binds", "specific for", when applied to an antibody, indicate that the antibody preferentially recognizes and/or binds the target polypeptide or epitope, i.e. with a higher affinity than to any other antigen or epitope, i.e. the binding to the target polypeptide can be discriminated from non-specific binding to other antigens. The binding affinity of an antibody can be readily determined by one of ordinary skill in the art, for example, by Scatchard analysis (Scatchard et al., 1949, Ann. N.Y. Acad. 1949. 51, 660-672*).*

As used herein, "binding affinity" generally refers to the apparent association constant or "Ka". The Ka is the reciprocal of the dissociation constant "Kd". Binding affinity may be determined by a variety of methods including equilibrium dialysis, equilibrium binding, gel filtration, ELISA, surface plasmon resonance or spectroscopy (e.g. using a fluorescence assay). Exemplary conditions for evaluating binding affinity are in buffer (50 mM Tris-HCl, 150 mM NaCl, 5 mM CaCl₂ at pH 7.5). These techniques can be used to measure the concentrations of bound and free binding protein as a function of binding protein (or target) concentration. The concentration of bound binding protein ([Bound]) is related to the concentration of free binding protein ([Free]) and the concentration of binding sites for the binding protein on the target where (N) is the number of binding sites per target molecule by the following equation: [Bound]=N x ([Free])/((1/Ka) + [Free]). Comparison of affinity between two antibodies can be established without actually determining the Ka value for each antibody, but based on a quantitative measurement of affinity (e.g. by ELISA or FACS analysis) that is proportional to Ka or a qualitative measurement of affinity or an inference of affinity (e.g. in functional assay or *in vitro* or *in vivo* assay).

The term "blocking" or "neutralizing" activity of an antibody refers to its ability to inhibit its target's activity. Applied to an antibody binding to MMP9, this term refers to the antibody's ability to generally neutralize MMP9 activity, by inhibiting activation of proMMP9 and/or by inhibiting the catalytic activity of activated MMP9 on one of its substrate such as gelatin, for instance as described in the example section. The neutralizing activity of an antibody may be determined by *in vitro* assays as described in the example section, or *in vivo* assays or functional assays such as a neutrophil/macrophages transmigration assay or a trans-epithelial electric resistance assay. A neutrophil transmigration (transwell) assay system allows addressing the ability of the neutrophils to produce active MMP9, degrade ECM and migrate through an endothelial cell monolayer, thus mimicking the *in vivo* process of neutrophil extravastion from blood into the submucosa, in response to proinflammatory chemokines. A trans-epithelial electric resistance (TEER) assay allows determining the effect of anti-MMP9 antibodies on the integrity and function of a reconstituted intestinal epithelial cell monolayer in response to cytokine-mediated disruption of the monolayer. The "potency" of an antibody may be expressed as the concentration of antibody/antigen-binding fragment which produces the half-maximal effect at a given antigen concentration. For example, the "effect" of an antibody may be inhibition or neutralization of its target's activity. In this case, the antibody concentration producing the half-maximal inhibition may be referred to as IC₅₀, which is given in mol/l or M. Potency is usually influenced by affinity until, at a given antigen concentration, an affinity is reached beyond which further improvements in affinity will not enhance binding of the antigen anymore (so-called potency ceiling). Applied to an antibody against MMP9, potency may, for example, be determined by measuring the IC₅₀ value of MMP9 dependent digestion of gelatin substrate in presence of the antibody.

The term "efficacy of inhibition" or "efficacy of neutralization", applied to a neutralizing antibody, is a measure of effectiveness of said antibody in inhibiting a specific biological or biochemical function. Total inhibition of the biological or biochemical activity is normalized to 100%. Applied to an antibody binding to MMP9, 100% efficacy may, for example, be antibody-mediated total inhibition of MMP9 dependent digestion of gelatin substrate.

The term "effector function" as used herein includes a biochemical event that results from the interaction of an antibody Fc region with an Fc receptor or ligand. Effector functions include FcγR-mediated effector functions such as ADCC (antibody dependent cell-mediated cytotoxicity) and ADCP (antibody dependent cell-mediated phagocytosis), and complement- mediated effector functions such as CDC (complement dependent cytotoxicity). An effector function of an antibody may be altered by altering, i.e. enhancing or reducing, preferably enhancing, the affinity of the antibody for an effector molecule such as an Fc receptor or a complement component. Binding affinity of an antibody Fc region with an Fc receptor or ligand can be altered by modifying the effector molecule binding site. It is also possible that an alteration in the binding site on the antibody for the effector molecule alters the geometry of the interaction without significantly altering the overall binding affinity, rendering the effector mechanism ineffective as in non-productive binding. It is also possible to alter an effector function by modifying a site not directly involved in effector molecule binding, but otherwise involved in performance of the effector function. By altering an effector function of an antibody it may be possible to control various aspects of the immune response, e.g. enhancing or suppressing various reactions of the immune system, with possible beneficial effects in diagnosis and therapy.

The term "pharmaceutically acceptable" refers to a carrier comprised of a material that is not biologically or otherwise undesirable.

The term "carrier" refers to any components present in a pharmaceutical formulation other than the active agent and thus includes diluents, binders, lubricants, disintegrants, fillers, coloring agents, wetting or emulsifying agents, pH buffering agents, preservatives and the like.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it for example based on familial history; (b) inhibiting the disease, i.e., arresting its development; or (c) relieving the disease, i.e., causing regression of the disease and/or its symptoms or conditions such as improvement or remediation of damage. For instance, treatment of inflammatory bowel disease comprises preventing, decreasing or even eradicating the symptoms of the diseases or disorders, for instance partial or total alleviation of diarrhea, abdominal pain and cramping, blood in the stool, abscess, ulcers and fistulas.

"MMP9-related diseases", as defined herewith, designate diseases mediated or influenced, at least in part, by the expression and/or activity of MMP9. Examples of MMP9-related diseases include inflammatory and autoimmune diseases, cancers or tumors, lung diseases, fibrotic lung diseases, septicemia, muscular dystrophy, allergy, renal fibrosis, scleroderma, dilated cardiomyopathy, Chagas disease, cardiovascular diseases, neuropsychiatric disorders, diabetes, and eye diseases.

The terms "inflammatory and autoimmune diseases" are generally defined herewith as inflammatory abnormalities which may or may not involve the immune system and diseases or disorders arising from an abnormal immune response of the subject's body against substances and tissues normally present in the body, respectively. Non-limitative examples of inflammatory and autoimmune diseases include mostly inflammatory bowel diseases (IBD) including Crohn's disease (CD) (in particular penetrating and stricturing Crohn's disease), ulcerative colitis (UC), indeterminate colitis, collagenous colitis, rheumatoid arthriris (RA), multiple sclerosis (MS), systemic lupus erythematosus (SLE), Sjogren's syndrome, systemic sclerosis, polymyositis, atherosclerosis.

The term "cancers" haematopoetic cancer, brain cancer, breast cancer, colorectal cancer, head and neck cancer, pancreatic cancer, lung cancer, liver cancer, melanoma, prostate cancer, muscle cancer, mesenchymal cancer, esophagogastric adenocarcinoma, non-small lung cancer, lung squamous cell carcinoma, lung adenocarcinoma, gastric adenocarcinoma, pancreatic adenocarcinoma, hepathocellular carcinomacolorectal cancer and hepathocellular carcinoma, in particular colorectal cancer and adenocarcinoma, in particular colorectal cancer and adenocarcinoma.

The term "subject" as used herein refers to mammals. For example, mammals contemplated by the present invention include human, primates, domesticated animals such as cattle, sheep, pigs, horses, laboratory rodents and the like.

The term "efficacy" of a treatment or method according to the invention can be measured based on changes in the course of disease or condition in response to a use or a method according to the invention. For example, the efficacy of a treatment or method according to the invention can be measured by its impact on signs or symptoms of illness. A response is achieved when the patient experiences partial or total alleviation, or reduction of unwanted symptoms of illness.

The term "effective amount" as used herein refers to an amount of at least one antibody according to the invention, or a pharmaceutical formulation thereof, that elicits a detectable reduction of the symptoms of the disease in a subject that is being administered said antibody, these symptoms can include, for instance: a) diarrhea, abdominal pain and cramping, blood in the stool, abscess, ulcers and fistulas, in the case of inflammatory bowel disease or b) constipation or diarrhea, bright red or dark red blood in stools, weight loss, fatigue, nausea and anemia, in the case of colorectal cancer.

### MMP9 binding proteins

### General characteristics of the MMP9 binding proteins

In a first aspect, the present invention provides proteins that bind to MMP9, in particular human MMP9, or a fragment thereof, and comprise at least one fragment of a heavy chain variable region and/or at least one fragment of a light chain variable region of an antibody as described herewith.

In one embodiment of the invention are provided isolated antibodies specific for MMP9, in particular human MMP9, or antigen-binding fragments thereof, comprising at least one fragment of a heavy chain variable region and at least one fragment of a light chain variable region, and optionally at least one fragment of a constant region, as described herewith.

The protein to which the antibodies according to the invention, or fragments thereof, bind can be the MMP9 protein of any species.

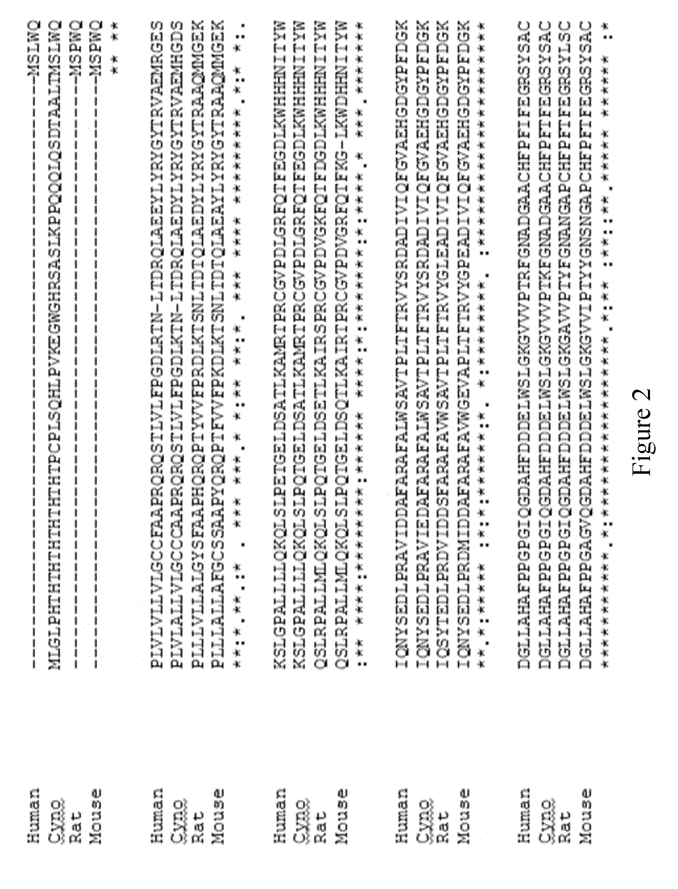

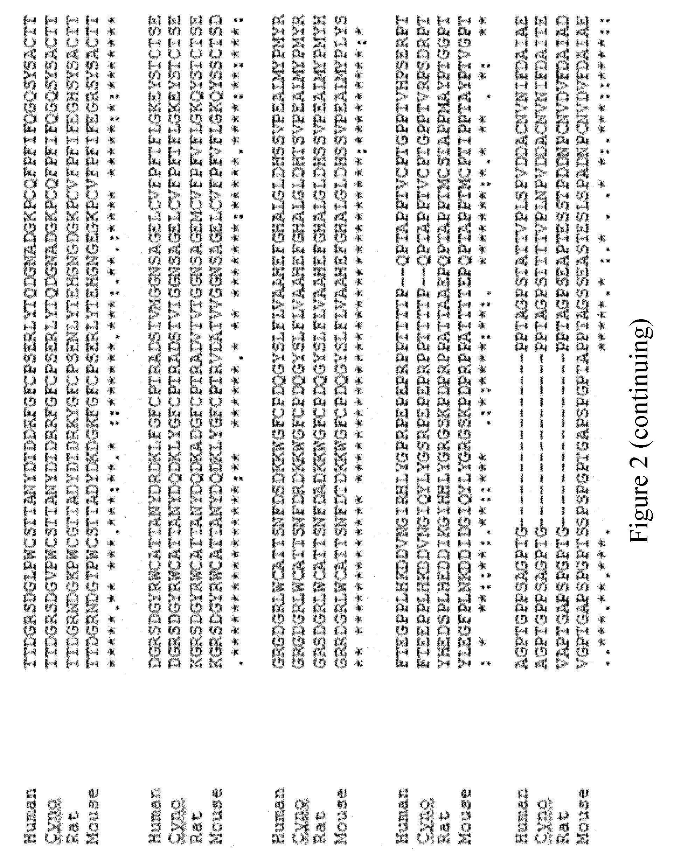

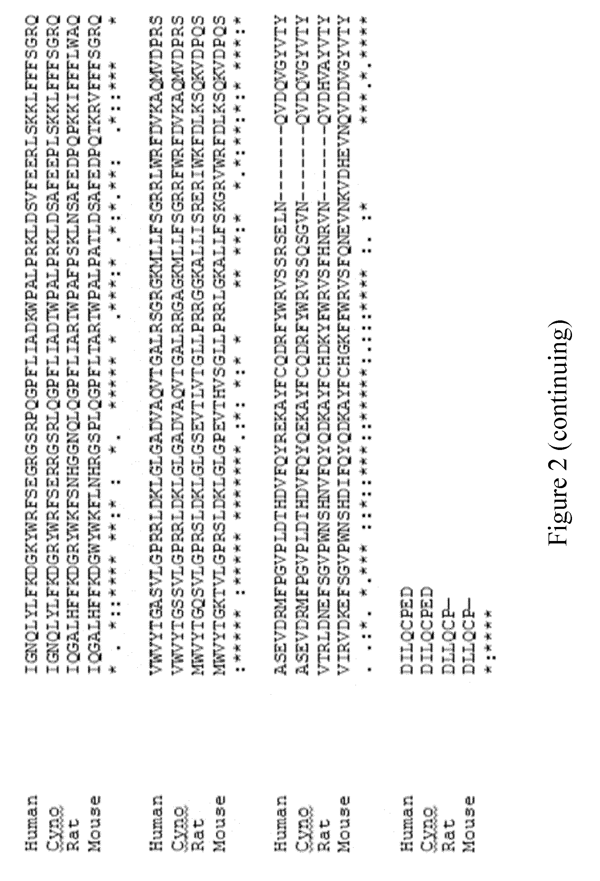

The antibodies according to the present invention generally exhibit a high specificity for human MMP9. However, depending on the degree of sequence identity between MMP9 homologs of different species (see Figure 2), a given antibody or antigen-binding fragment may show cross-reactivity with MMP9 from at least one other species, e.g. mouse, rat, marmoset, monkey (e.g. Cynomologus monkey), dog, and/or rabbit. For antibodies directed towards human MMP9, some level of cross-reactivity with other mammalian forms of MMP9 may be desirable in certain circumstances, for example when testing antibodies in animal models of a particular disease or for conducting toxicology, safety and dosage studies.

In a specific embodiment, the antibodies according to the invention or fragments thereof bind preferentially to human MMP9.

In another embodiment, the antibodies according to the invention or fragments thereof show cross-reactivity with human MMP9, Cynomologus monkey MMP9, rat MMP9 and, optionally, mouse MMP9.

In some embodiments, the binding affinity (e.g. inversely correlated to the Kd value) of antibodies, and fragments thereof, according to the invention for human MMP9 is at least 2 times, at least 5 times, at least 10 times, at least 50 times, at least 100 times, at least 500 times, or at least 1000 times higher than their binding affinity for a non-human MMP9.

In one embodiment, the antibodies according to the invention or fragments thereof bind preferentially to MMP9 and, optionally, additionally exhibit a weak binding, or virtually no binding (i.e. negligible or not detectable binding) to other matrix metalloproteinases (MMPs) such as MMP1, MMP2, MMP3, MMP8, MMP9, MMP10, MMP12, MMP13, MMP14, MMP19.

In a particular embodiment, the antibodies according to the invention, or fragments thereof, bind preferentially to MMP9 and exhibit a weak, or virtually no (i.e. negligible or not detectable), binding to MMP2.

For therapeutic uses, it may be advantageous that the antibodies according to the invention, or fragments thereof, do not bind, and thus do not neutralize, MMP2 so as not to substantially affect MMP2 activity. Indeed, MMP2 is required for normal tissue homeostasis and may also have a protective role against disease as suggested by observations according to which MMP2 knockout mice show a worsen phenotype than wild-type mice, in several disease models.

In some embodiments, the binding affinity of antibodies (e.g. inversely correlated to the Kd value), and fragments thereof, according to the invention for MMP9 is at least 2 times, at least 5 times, at least 10 times, at least 50 times, at least 100 times, at least 500 times, or at least 1000 times higher than their binding affinity for MMP2.

Binding affinity can be measured by any method known in the art including equilibrium dialysis, equilibrium binding, gel filtration, ELISA, surface plasmon resonance or spectroscopy (e.g. using a fluorescence assay) *(*Jiang et al. BMC Pharmacology 2010, 10:10*)* and can be expressed as, for instance, on-rate, off-rate, dissociation constant (Kd), equilibrium constant (Keq) or any other term used in the art.

In some embodiments, the antibodies, and fragments thereof, according to the invention specifically bind to human MMP9 with a dissociation constant (Kd) equal to or lower than 100 nM, in particular lower than 10 nM, more particularly lower 1 nM, or lower than 0.5 nM, or lower than 0.1 nM, or lower than 0.01 nM, or lower than 0.005 nM. The protein to which the antibodies according to the invention, or fragments thereof, bind to is any form of MMP9: the immature protein comprising the secretory leader sequence ("preproenzyme") (corresponding to residues 1-707 of SEQ ID NO: 1 in the case of human MMP9), the mature latent MMP9 lacking the secretory leader sequence ("proenzyme") (corresponding to residues 20-707 of SEQ ID NO: 1 in the case of human MMP9), the "activated enzyme" (corresponding to residues 107-707 of SEQ ID NO: 1 in the case of human MMP9), or any fragment of MMP9.

The antibodies according to the invention, or fragments thereof, can bind to MMP9 by interacting with an epitope comprising amino acids located anywhere in the protein, e.g. in the prodomain, the catalytic domain, in particular in the Fn-repeats or the OG domain linker, the recognized amino acids being located at one or more sites within the protein. Therefore, in one embodiment, the antibodies according to the invention, or fragments thereof, not only bind to MMP9 but also neutralize or inhibit MMP9 activity (e.g. MMP9 catalytic activity) by inhibiting processing of the preproenzyme and/or proenzyme to the catalytically active enzyme and/or by inhibiting the proteolytic activity of the activated enzyme.

In a particular embodiment, the isolated antibody or fragment thereof according to the invention inhibits the catalytic activity of MMP9.

In another particular embodiment, the isolated antibody or fragment thereof according to the invention inhibits processing of the preproenzyme MMP9 or the proenzyme MMP9 to the catalytically active MMP9

In a particular embodiment, the antibodies according to the present invention exhibit a high specificity and inhibitory activity for human MMP9 and may show cross-reactivity with Cynomologus monkey MMP9, rat MMP9, and/or mouse MMP9.

In a particular embodiment, the antibodies according to the invention or fragments thereof inhibit MMP9 activity and, optionally, additionally exhibit a weak inhibitory activity, or virtually no inhibitory activity (i.e. negligible or not detectable activity) towards other matrix metalloproteinases (MMPs) such as MMP1, MMP2, MMP3, MMP8, MMP9, MMP10, MMP12, MMP13, MMP14, MMP19.

In a particular embodiment, the antibodies according to the invention, or fragments thereof, exhibit a neutralizing activity towards MMP9 and a weak, or virtually no (i.e. negligible or not detectable), neutralizing activity towards MMP2.

The ability of an antibody to block or neutralize the activity of its target protein can be evaluated by its potency as defined herewith, which is itself reflected, for instance, by the IC₅₀ value. Typically, the neutralizing activity of an antibody may be determined by *in vitro* assays, such as an assay measuring the catalytic activity of MMP9 towards gelatin as described in the example section, *in vivo* assays or functional assays such as an assay measuring the catalytic activity of MMP9 towards gelatin, a neutrophil/macrophage transmigration assay or a trans-epithelial electric resistance assay.

A neutrophil transmigration (transwell) assay system allows addressing the ability of the neutrophils to produce active MMP9, degrade ECM and migrate through an endothelial cell monolayer, thus mimicking the *in vivo* process of neutrophil extravastion from blood into the submucosa, in response to proinflammatory chemokines. A trans-epithelial electric resistance (TEER) assay allows determining the effect of anti-MMP9 antibodies on the integrity and function of a reconstituted intestinal epithelial cell monolayer in response to cytokine-mediated disruption of the monolayer. In some embodiments, the inhibitory or neutralizing potency (e.g. inversely correlated to the IC₅₀ value) of antibodies, and fragments thereof, according to the invention for human MMP9 is at least 2 times, at least 5 times, at least 10 times, at least 50 times, at least 100 times, at least 500 times, or at least 1000 times higher than their neutralizing potency for non-human MMP9.

In some embodiments, the inhibitory or neutralizing potency e.g. inversely correlated to the IC₅₀ value, of antibodies, and fragments thereof, according to the invention on MMP9 is at least 10 times, at least 50 times, at least 100 times, at least 500 times, or at least 1000 times higher than their inhibitory or neutralizing potency on MMP2.

In some embodiments, the antibodies, and fragments thereof, according to the invention have a IC₅₀ equal to or lower than 200 nM, in particular lower than 100 nM, in particular lower than 50 nM, more particularly lower than 10 nM, lower than 8 nM, lower than 7 nM, lower than 5 nM, lower than 3 nM, lower than 2 nM, lower than 1 nM, lower than 0.5 nM, lower than 0.3 nM, lower than 0.2 nM, or lower than 0.1 nM; for inhibiting MMP9 catalytic activity on gelatin.

The ability of an antibody to block or neutralize the activity of its target protein can also be evaluated by its efficacy of inhibition as defined herewith, which is itself reflected, for instance, by the percentage (%) of inhibition value.

In some embodiments, the antibodies, and fragments thereof, according to the invention have an efficacy equal to or superior than 50%, in particular equal to or superior than 60%, in particular equal to or superior than 70%, in particular equal to or superior than 80%, in particular equal to or superior than 90%, in particular equal to or superior than 95%, in particular equal to 100%, for inhibiting processing of the preproenzyme and/or proenzyme and/or by inhibiting the proteolytic activity of the activated MMP9, as determined for instance in an assay measuring the catalytic activity of MMP9 towards gelatin as described in the example section.

It is understood that any variant of an antibody according to the invention, or fragment thereof, that is described herewith is able to bind MMP9 and optionally neutralize MMP9 activity. In a particular embodiment, such variant can show the same or even higher binding affinity for MMP9 and/or the same or even higher potency and/or the same or greater species-selectivity and/or the same or greater selectivity for MMP9, and/or the same or greater neutralizing efficacy, in comparison to the parental antibody or fragment from which said variant derives.

The antibodies according to the invention can be monoclonal antibodies, polyclonal antibodies, human antibodies, humanized antibodies, chimeric antibodies, and further engineered antibodies as long as the characteristic properties of the invention are retained, in particular the ability of binding to the target antigen, more specifically to the same epitope of MMP9 as the one recognized by the antibodies of the invention, and optionally the ability of neutralizing MMP9 activity.

In a particular embodiment of the invention, the antibodies specific for MMP9 according to the invention, or fragments thereof which specifically bind to MMP9, are monoclonal antibodies.

In a further particular embodiment of the invention, the antibodies specific for MMP9 according to the invention, or fragments thereof which specifically bind to MMP9, are human antibodies.

The antibodies specific for MMP9 according to the invention, or fragments thereof which specifically bind to MMP9, can be characterized by their portion interacting with the target's protein, in particular by their variable region, which typically comprises a heavy chain variable region and a light chain variable region.

### Characteristics of the MMP9 binding proteins in relation to their heavy chain variable region

In one embodiment, the invention relates to isolated antibodies specific for MMP9 or antigen-binding fragments thereof comprising a heavy chain variable region comprising:
(i) a heavy chain CDR1 of SEQ ID NO: 2 or a variant thereof wherein 1, 2, or 3 amino acids of said heavy chain CDR1 is substituted by a different amino acid;
(ii) a heavy chain CDR2 of SEQ ID NO: 3 or a variant thereof wherein 1, 2, or 3 amino acids of said heavy chain CDR2 is substituted by a different amino acid;
(iii) a heavy chain CDR3 of SEQ ID NO: 4 or a variant thereof wherein 1, 2, or 3 amino acids of said heavy chain CDR3 is substituted by a different amino acid.

In a particular embodiment of the invention, at least one amino acid at positions 50 and 55 of said heavy chain CDR2 is substituted by a different amino acid, in particular with at least one of the following substitutions: W50Y and G55A.

In another particular embodiment of the invention, said heavy chain CDR2 variant has an amino acid sequence selected among: SEQ ID NO: 5, SEQ ID NO: 6, and SEQ ID NO: 7.

In another particular embodiment of the invention, the antibodies specific for MMP9, or antigen-binding fragments thereof, comprise a heavy chain variable region of SEQ ID NO: 8 or SEQ ID NO: 9, or a variant thereof wherein 1, 2, 3, 4, 5, 6, 7, 8, 9, 10, 15, 20 and up to 23 amino acids of SEQ ID NO: 8 or SEQ ID NO: 9 is substituted by a different amino acid, or a variant thereof having at least 80%, at least 85%, at least 90%, at least 95%, at least 98%, at least 99% identity with SEQ ID NO: 8 or SEQ ID NO: 9, respectively.

In a further embodiment, the antibodies specific for MMP9, or antigen-binding fragments thereof, comprise a variant of SEQ ID NO: 8 or SEQ ID NO: 9, wherein 1, 2, or 3 amino acids of at least one of the heavy chain CDR1, CDR2, and/or CDR3, and/or 1, 2, 3, 4, 5, 6, 7, 8, 9, 10, 11, 12, 13,14 or 15 amino acids of the heavy chain variable framework region is substituted by a different amino acid.

In particular, the antibodies specific for MMP9 or fragment thereof according to the invention comprise a variant of SEQ ID NO: 8, wherein at least one amino acid at positions 50 and 55 of the heavy chain CDR2 and/or at least one amino acid at positions 1, 6, 76 and 79 of the heavy chain variable framework region is substituted by a different amino acid, in particular with at least one of the following substitutions: W50Y, G55A, Q1E, E6Q, N76S, H79Y.

More particularly, the antibodies specific for MMP9 or fragment thereof according to the invention comprise a variant of SEQ ID NO: 8, wherein amino acids at positions 1, 6, 76 and 79 of the heavy chain variable framework region and at least one amino acid at positions 50 and 55 of the heavy chain CDR2 is substituted by a different amino acid, in particular with Q1E, E6Q, N76S, H79Y substitutions and at least one of the following substitutions: W50Y and G55A.

Specific examples of the heavy chain variable region comprised in the antibodies or fragments thereof according to the invention include:
(i) the amino acid sequence SEQ ID NO: 8,
(ii) the amino acid sequence SEQ ID NO: 9,
(iii) the amino acid sequence SEQ ID NO: 10,
(iv) the amino acid sequence SEQ ID NO: 11,
(v) the amino acid sequence SEQ ID NO: 12.

In particular, the heavy chain variable region comprised in the antibodies or fragments thereof according to the invention includes the amino acid sequence selected from: SEQ ID NO: 9, SEQ ID NO: 10, and SEQ ID NO: 12.

### Characteristics of the MMP9 binding proteins in relation to their light chain variable region

In one embodiment, the invention relates to isolated antibodies specific for MMP9 or antigen-binding fragments thereof comprising a heavy chain variable region as described above and further comprising a light chain variable region comprising:
(i) a light chain CDR1 of SEQ ID NO: 13 or a variant thereof wherein 1, 2, or 3 amino acids of said light chain CDR1 is substituted by a different amino acid;
(ii) a light chain CDR2 of SEQ ID NO: 14 or a variant thereof wherein 1, 2, or 3 amino acids of said light chain CDR2 is substituted by a different amino acid;
(iii) a light chain CDR3 of SEQ ID NO: 15 or a variant thereof wherein 1, 2, or 3 amino acids of said light chain CDR3 is substituted by a different amino acid.

In a particular embodiment of the invention, the antibodies specific for MMP9, or antigen-binding fragments thereof, comprise a light chain variable region of SEQ ID NO: 16 or SEQ ID NO: 17, or a variant thereof wherein 1, 2, 3, 4, 5, 6, 7, 8, 9, 10, 15, up to 20 or up to 21 amino acids of SEQ ID NO: 16 or SEQ ID NO: 17 is substituted by a different amino acid, or a variant thereof having at least 80%, at least 85%, at least 90%, at least 95%, at least 98%, at least 99% identity with SEQ ID NO: 16 or SEQ ID NO: 17, respectively.

In a further embodiment, the antibodies specific for MMP9, or antigen-binding fragments thereof, comprise a variant of SEQ ID NO: 16 or SEQ ID NO: 17, wherein 1, 2, or 3 amino acids of at least one of the light chain CDR1, CDR2, and/or CDR3, and/or 1, 2, 3, 4, 5, 6, 7, 8, 9, 10, 11, 12, 13, 14 or 15 amino acids of the light chain variable framework region is substituted by a different amino acid.

In particular, the antibodies specific for MMP9 or fragment thereof according to the invention comprise a variant of SEQ ID NO: 16, wherein at least one amino acid at positions 10, 72, 74, 77, 78, 79, 80, 81, and 84 of the light chain variable framework region is substituted by a different amino acid, in particular with at least the following substitution: T10A, T72S, T74A, R77G, V78L, E79Q, V80S, G81E, G84A.

One specific example of the variable region comprised in the antibodies or fragments thereof according to the invention includes the amino acid sequence SEQ ID NO: 17.

### Characteristic of the MMP9 binding proteins in relation to their constant region

A portion corresponding to a constant region of an antibody is optionally comprised in the isolated antibodies specific for MMP9, or antigen-binding fragments thereof, according to the invention.

Depending on the proposed function of the antibodies and, in particular the effector functions which may be required, a constant region of an antibody may or may not be present within the antibodies according to the invention.

Typically, when present within the antibodies or antigen-binding fragments thereof according to the invention, the heavy chain constant region or portion thereof can be from any antibody isotype. For instance, the heavy chain constant region or portion thereof can be that of an antibody selected from IgG (e.g. IgG1, IgG2, IgG3, IgG4), IgA (e.g. IgA1, IgA2), IgD, IgE, IgM (e.g. IgM1, IgM2). It can be, in particular, the constant region or portion thereof of an IgG, more particularly IgG4.

In particular, human IgG constant region domains may be used, especially of the IgG1 and IgG3 isotypes when the antibody molecule is intended for therapeutic uses and antibody effector functions are required. Alternatively, IgG2 and IgG4 isotypes may be used when the antibody molecule is intended for therapeutic purposes and antibody effector functions are not required, e.g. for simply blocking MMP9 activity.

It will be appreciated that sequence variants of these constant region domains may also be used. For instance, the heavy chain constant region or portion thereof can be that of an engineered variant of IgG4 such as an IgG4 variant comprising S228P, R409K and a deletion of the terminal lysine, corresponding to amino acid sequence SEQ ID NO: 26. When present within the antibodies or antigen-binding fragments thereof according to the invention, the light chain constant region or portion thereof can be from any light chain's constant region. For instance, the light chain constant region or portion thereof can be from the kappa or lambda light chain.

In a particular aspect of the invention, the antibodies specific for MMP9, or antigen-binding fragments thereof, comprise (i) at least one heavy chain comprising a variable region as described herewith and a constant region or portion thereof from an IgG antibody, and (ii) at least one light chain comprising a variable region as described herewith and a constant region or portion thereof from a lambda (in particular lambda 2) light chain.

In a particular embodiment, the constant region or portion thereof of the heavy chain and/or of the light chain, which is comprised in the antibodies according to the invention, has an amino acid sequence which has been modified compared to its original amino acid sequence, according to methods known in the art, to increase the chemical stability of the antibodies, decrease their aggregation, increase their production in particular in antibody-producing cells (e.g. HEK293 cells, CHO cells), and/or eliminate their ability to exchange half-molecules which would effectively result in monovalent antibodies.

Examples of amino acids within the amino acid sequence of the constant region of an antibody which affect the antibody's stability include S228P amino acid mutation (EU numbering) in the human IgG4 heavy chain that stabilizes the hinge domain of the antibody (Angal et al, 1993, Molec. Immunol. 30: 105-108*)* and avoids Fc-Fc interactions *(*Rispens et al., 2013, Mol. Immunol. 53: 35-42*),* a Lys (K) rather than an Arg (R) at allotypic position 409 (EU numbering) that increases the CH3-CH3 interaction strength in IgG4 (Allberse et al, 2002, Immunology 105: 9-19*),* those examples are incorporated herewith by reference. In addition, in order to simplify the monitoring of monoclonal antibody charge heterogeneity, the C-terminal lysine of the human IgG4 heavy chain can be removed.

In a further embodiment, the antibodies specific for MMP9, or antigen-binding fragments thereof, according to the invention comprise at least one heavy chain comprising a variable region as described herewith and a constant region or portion thereof from an IgG4 antibody, wherein the amino acid sequence of the IgG4 constant region comprises the following amino acids modifications: S228P (EU numbering), R409K (EU numbering), deletion of the terminal Lys (K), wherein said modified constant region is represented by SEQ ID NO: 26.

It derives from the above that the present invention provides, in particular, isolated antibodies specific for MMP9 or antigen-binding fragment thereof comprising:
(1) a heavy chain variable region comprising:
   (i) a heavy chain CDR1 of SEQ ID NO: 2 or a variant thereof wherein 1, 2, or 3 amino acids of said heavy chain CDR1 is substituted by a different amino acid;
   (ii) a heavy chain CDR2 of SEQ ID NO: 3 or a variant thereof wherein 1, 2, or 3 amino acids of said heavy chain CDR2 is substituted by a different amino acid;
   (iii) a heavy chain CDR3 of SEQ ID NO: 4 or a variant thereof wherein 1, 2, or 3 amino acids of said heavy chain CDR3 is substituted by a different amino acid; and
(2) a light chain variable region comprising:
   (i) a light chain CDR1 of SEQ ID NO: 13 or a variant thereof wherein 1, 2, or 3 amino acids of said light chain CDR1 is substituted by a different amino acid;
   (ii) a light chain CDR2 of SEQ ID NO: 14 or a variant thereof wherein 1, 2, or 3 amino acids of said light chain CDR2 is substituted by a different amino acid;
   (iii) a light chain CDR3 of SEQ ID NO: 15 or a variant thereof wherein 1, 2, or 3 amino acids of said light chain CDR3 is substituted by a different amino acid.

In another particular embodiment of the invention, said heavy chain CDR2 variant has an amino acid sequence selected among: SEQ ID NO: 5, SEQ ID NO: 6, and SEQ ID NO: 7.

The antibodies of the invention have at least one antigen binding site, e.g. one or two antigen binding sites. In certain embodiments, for instance for CDR-comprising peptides, a variable domain may contain only CDRs linked via short linker peptides instead of complete framework regions.

In some embodiments, the isolated antibodies and antigen-binding fragments thereof according to the invention are glycosylated. Typically, monosaccharides such as N-acetylglucosamine, mannose, glucose, galactose, fucose, sialic acid, etc, are assembled to oligosaccharides at individual glycosylation sites on the antibody.

### Examples of antibodies specific for MMP9 or antigen-binding fragment thereof

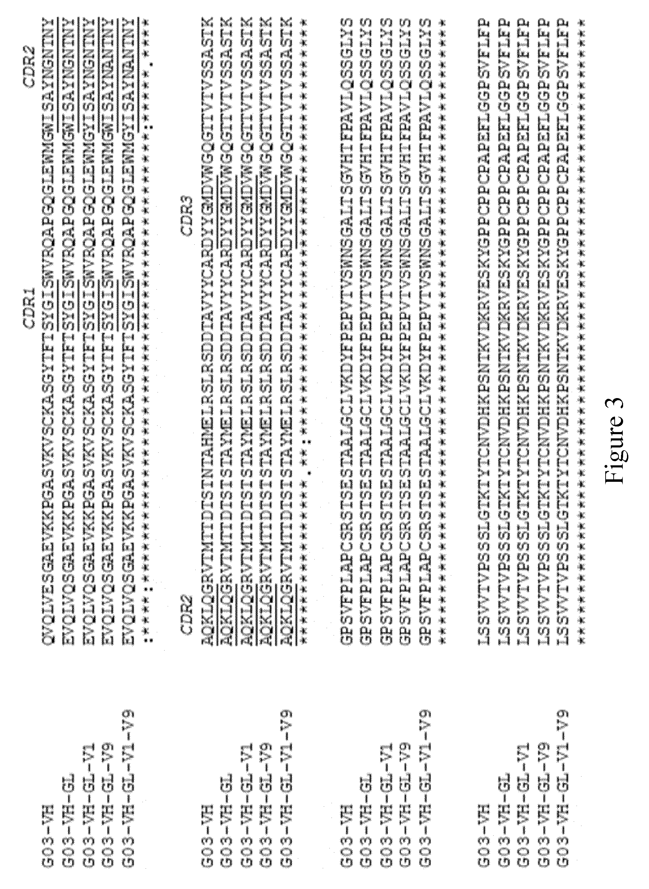

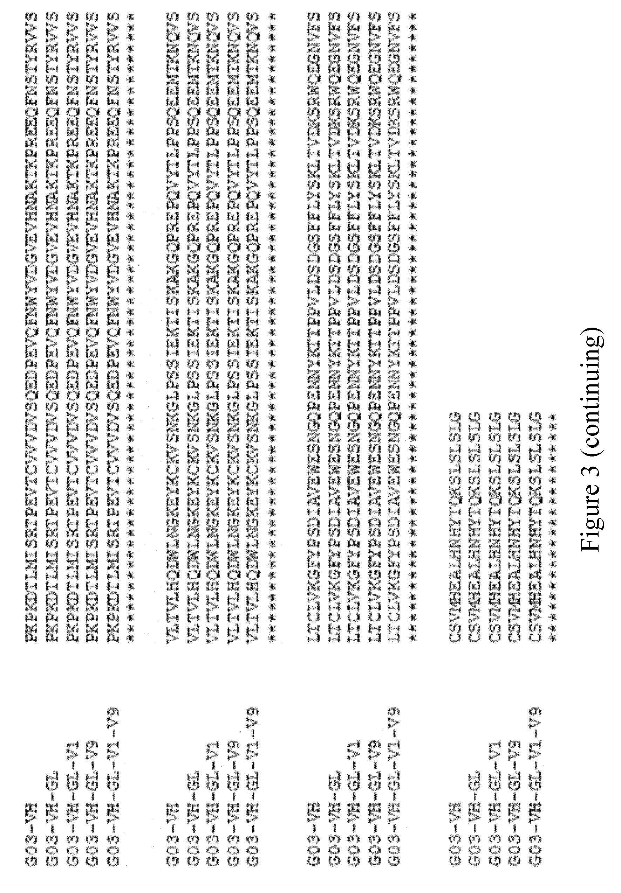

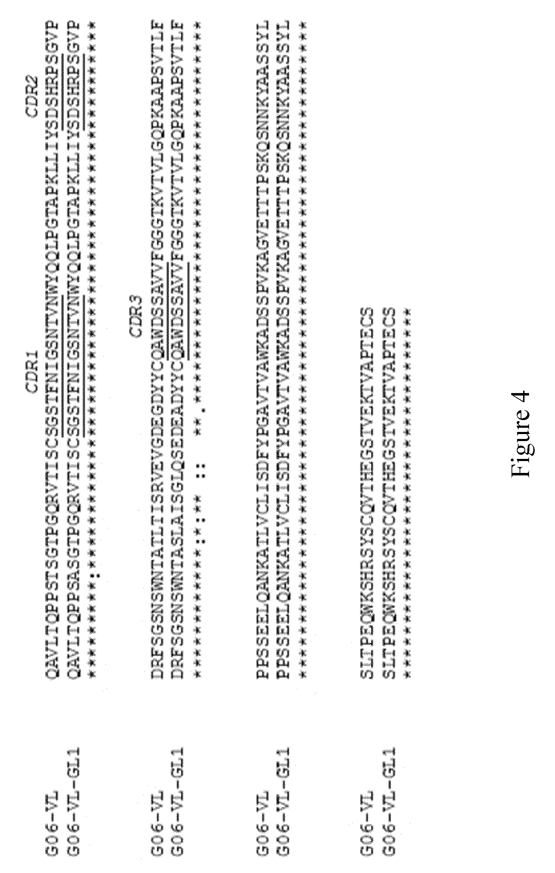

Examples of antibodies and fragments thereof according to the invention include those comprising the variable domains indicated in Table 2 (see also Figures 3 and 4).

**Table 2**

| **Name** | **Sequence of Heavy chain variable region** | **Sequence of Light chain variable region** |
|---|---|---|
| G03-VH / G06-VL | SEQ ID NO: 8 | SEQ ID NO: 16 |
| G03-VH-GL / G06-VL | SEQ ID NO: 9 | SEQ ID NO: 16 |
| G03-VH-GL-V1 / G06-VL | SEQ ID NO: 10 | SEQ ID NO: 16 |
| G03-VH-GL-V9 / G06-VL | SEQ ID NO: 11 | SEQ ID NO: 16 |
| G03-VH-GL-V1-V9 / G06-VL | SEQ ID NO: 12 | SEQ ID NO: 16 |
| G03-VH / G06-VL-GL1 | SEQ ID NO: 8 | SEQ ID NO: 17 |
| G03-VH-GL / G06-VL-GL1 | SEQ ID NO: 9 | SEQ ID NO: 17 |
| G03-VH-GL-V1 / G06-VL-GL1 | SEQ ID NO: 10 | SEQ ID NO: 17 |
| G03-VH-GL-V9 / G06-VL-GL1 | SEQ ID NO: 11 | SEQ ID NO: 17 |
| G03-VH-GL-V1-V9 / G06-VL-GL1 | SEQ ID NO: 12 | SEQ ID NO: 17 |

Thus, in a specific embodiment, MMP9 binding proteins according to the invention include isolated antibodies specific for MMP9 or antigen-binding fragments thereof, comprising:
(1) a heavy chain variable region selected among:
   (i) the amino acid sequence of SEQ ID NO: 8,
   (ii) the amino acid sequence of SEQ ID NO: 9,
   (iii) the amino acid sequence of SEQ ID NO: 10,
   (iv) the amino acid sequence of SEQ ID NO: 11,
   (v) the amino acid sequence of SEQ ID NO: 12,
(2) a light chain variable region selected among:
   (i) the amino acid sequence of SEQ ID NO: 16,
   (ii) the amino acid sequence of SEQ ID NO: 17.

In a more particular embodiment, MMP9 binding proteins according to the invention include isolated antibodies specific for MMP9 or antigen-binding fragments thereof, comprising:
(1) a heavy chain variable region selected among:
   (i) the amino acid sequence of SEQ ID NO: 9,
   (ii) the amino acid sequence of SEQ ID NO: 10,
   (iii) the amino acid sequence of SEQ ID NO: 12, and
(2) a light chain variable region of amino acid sequence SEQ ID NO: 17.

### Conjugates comprising an auxiliary molecule

In another aspect of the invention, the isolated antibodies or antigen-binding fragment thereof according to the invention are optionally conjugated to an accessory molecule, and are then also referred to herein as "conjugated antibodies or "conjugated antibody fragments".

The accessory molecule may be conjugated to the antibody or antibody fragment directly or via a spacer of suitable length for instance as described in Kellogg et al. (2011, Bioconjug Chem, 22: 717-27).

In one embodiment, particularly adapted for therapeutic purposes, the accessory molecule can be a therapeutic effector group such as a cytotoxic (e.g. an enzymatically active toxin of bacterial, fungal, plant or animal origin, or fragment thereof), cytostatic, or immunomodulatory agent, including radioactive groups (i.e groups comprising a radionucleide or radioisotope), or small molecules.

In another embodiment, the accessory molecule comprises an antigen-binding fragment of an antibody, which, when conjugated to the antibody or antibody fragment according to the invention, form a bispecific antibody. In particular, said bispecific antibody may be directed to two different MMPs or to two different epitopes of one MMP such as two different epitopes of MMP9.

In a specific embodiment, the accessory molecule may be, for example, an agent active for the treatment of a disease, such as, for the treatment of Crohn's disease: the variable region of an anti-TNFα antibody, which, when conjugated to an antibody or antigen-binding fragment of the invention can form bi-specific anti-TNFα/MMP9 antibodies for the treatment of subjects suffering from moderate to severe Crohn's disease.

The conjugated antibodies and conjugated antibody fragments according to the invention can target the drug *in vivo* to a site of disease (e.g. a site of inflammation or a tumor) such that the conjugated auxiliary molecule can have a therapeutic effect on the site of disease.

In an alternative embodiment, particularly adapted for diagnostic purposes, the accessory molecule can be, for example, a labeling group including radioisotopes (e.g. 3H, 14C, 32P, 35S, I25I), chromogenic labels, e.g. enzymes which can be used to convert a substrate to a detectable colored (e.g. horseradish peroxidase, alkaline phosphatase, β-galactosidase) or fluorescent compound (e.g. Green Fluorescent Protein, Red Fluorescent Protein), spectroscopic labels (e.g. fluorescent labels such as fluorescein and its derivatives like FITC, Texas red, cyanine dyes, photocyan, rhodamine, or labels presenting a visible color), luminescent labels including luciferins, affinity labels which may be developed by a further compound specific for the label and allowing easy detection and quantification, or any other label used in standard ELISA.

### Nucleic acids encoding the polypeptides of the invention

According to another embodiment, it is provided an isolated nucleic acid molecule encoding an antibody or antigen-binding fragment thereof according to the invention.

The isolated nucleic acid according to the invention may be, for instance, natural DNA or RNA or a recombinant or synthetic DNA, RNA or LNA or a recombinant nucleic acid molecule comprising any of the nucleic acid molecules according to the invention either alone or in combination. In a particular embodiment, the nucleic acid molecules according to the invention are cDNA.

In a particular embodiment, it is provided an isolated nucleic acid comprising one or more of:
(1) a nucleic acid sequence encoding a heavy chain CDR1 of SEQ ID NO: 2, a heavy chain CDR2 of SEQ ID NO: 3, a heavy chain CDR3 of SEQ ID NO: 4, or a variant thereof wherein 1, 2, or 3 amino acids of said heavy chain CDR is substituted by a different amino acid, and
(2) a nucleic acid encoding a light chain CDR1 of SEQ ID NO: 13, a light chain CDR2 of SEQ ID NO: 14, a light chain CDR3 of SEQ ID NO: 15, or a variant thereof wherein 1, 2, or 3 amino acids of said light chain CDR is substituted by a different amino acid.

In a particular embodiment, it is provided an isolated nucleic acid comprising one or more of:
(1) a nucleic acid sequence encoding a heavy chain variable region selected among:
   (i) the amino acid sequence SEQ ID NO: 8,
   (ii) the amino acid sequence SEQ ID NO: 9,
   (iii) the amino acid sequence SEQ ID NO: 10,
   (iv) the amino acid sequence SEQ ID NO: 11,
   (v) the amino acid sequence SEQ ID NO: 12, and
(2) a nucleic acid sequence encoding a light chain variable region selected among:
   (i) the amino acid sequence SEQ ID NO: 16
   (ii) the amino acid sequence SEQ ID NO: 17.

In a more particular embodiment, the invention provides an isolated nucleic acid comprising one or more of:
(1) a nucleic acid sequence encoding a heavy chain variable region selected among:
   (i) the amino acid sequence SEQ ID NO: 9,
   (ii) the amino acid sequence SEQ ID NO: 10,
   (iii) the amino acid sequence SEQ ID NO: 12, and
(2) a nucleic acid sequence encoding a light chain variable region of amino acid sequence SEQ ID NO: 17.

In another particular embodiment, it is provided an isolated nucleic acid comprising one or more of:
(1) a nucleic acid sequence comprising SEQ ID NO: 18, SEQ ID NO: 19, and SEQ ID NO: 20, or a variant thereof having at least 80%, at least 90%, at least 95%, at least 96%, at least 97%, at least 98%, or at least 99% identity with one of said sequences, and/or
(2) a nucleic acid sequence comprising SEQ ID NO: 23, SEQ ID NO: 24, and SEQ ID NO: 25, or a variant thereof having at least 80%, at least 90%, at least 95%, at least 96%, at least 97%, at least 98%, or at least 99% identity with one of said sequences.

In a further particular embodiment, the variant of SEQ ID NO: 19 is selected among SEQ ID NO: 21 and SEQ ID NO: 22.

### Vectors and Host cells for production and purification of the polypeptides of the invention

In one embodiment, the invention provides a recombinant expression vector comprising a nucleic acid molecule according to the invention, wherein the vector optionally comprises an expression control sequence, allowing expression in prokaryotic or eukaryotic host cells of the encoded polypeptide, operably linked to said nucleic acid molecule.

Numerous expression systems can be used, including without limitation chromosomes, episomes, and derived viruses. More particularly, the recombinant vectors used can be derived from bacterial plasmids, transposons, yeast episomes, insertion elements, yeast chromosome elements, viruses such as baculovirus, papilloma viruses such as SV40, vaccinia viruses, adenoviruses, fox pox viruses, pseudorabies viruses, retroviruses. These recombinant vectors can equally be cosmid or phagemid derivatives.

The nucleic acid sequence can be inserted in the recombinant expression vector by methods well known to a person skilled in the art such as, for example, those that are described in MOLECULAR CLONING: A LABORATORY MANUAL, Sambrook et al., 4th Ed., Cold Spring Harbor Laboratory Press, Cold Spring Harbor, N.Y., 2001.

The recombinant vector can include nucleotide sequences that control the regulation of the polynucleotide expression as well as nucleotide sequences permitting the expression and the transcription of a polynucleotide of the invention and the translation of a polypeptide of the invention, these sequences being selected according to the host cells that are used.

Thus, for example, an appropriate secretion signal can be integrated in the recombinant vector so that the polypeptide, encoded by the nucleic acid molecule of the invention, will be directed towards the lumen of the endoplasmic reticulum, towards the periplasmic space, on the membrane or towards the extracellular environment. The choice of an appropriate secretion signal may facilitate subsequent protein purification. In a further embodiment, it is provided a host cell comprising a recombinant vector according to the invention.

The introduction of the recombinant vector in a host cell can be carried out according to methods that are well known to a person skilled in the art, such as those described in BASIC METHODS IN MOLECULAR BIOLOGY, Davis et al., 2nd ed., McGraw-Hill Professional Publishing, 1995, and MOLECULAR CLONING: A LABORATORY MANUAL, supra, such as transfection by calcium phosphate, transfection by DEAE dextran, transfection, microinjection, transfection by cationic lipids, electroporation, transduction or infection.

The host cell can be, for example, bacterial cells such as *E. coli* or *Streptomyces,* cells of fungi such as *Aspergillus* and yeasts such as *Saccharomyces,* insect cells, Chinese Hamster Ovary cells (CHO), C127 mouse cell line, BHK cell line of Syrian hamster cells, Human Embryonic Kidney 293 (HEK 293) cells. In a particular embodiment, the host cell is a CHO cell or a HEK 293 cell.

The host cells can be used, for example, to express a polypeptide of the invention. After purification by standard methods, the polypeptide of the invention can be used in a method described hereinafter.

For instance, when expression systems that secrete the recombinant protein are employed, the culture medium may first be concentrated using a commercially available protein concentration filter, for example, an Amicon or Millipore Pellicon ultrafiltration unit. Following the concentration step, the concentrate can be applied to a purification matrix such as a gel filtration matrix. Alternatively, an anion exchange and/or an affinity resin can be employed. The matrices can be acrylamide, agarose, dextran, cellulose or other types commonly employed in protein purification. Alternatively, a cation exchange step can be employed. Finally, one or more reversed-phase high performance liquid chromatography (RP-HPLC) steps employing hydrophobic RP-HPLC media can be employed to further purify the antibodies or fragments thereof. Some or all of the foregoing purification steps, in various combinations, are well known and can be employed to provide a substantially homogeneous recombinant protein. Recombinant protein produced in bacterial culture can be isolated by initial disruption of the host cells, centrifugation, extraction from cell pellets if an insoluble polypeptide, or from the supernatant fluid if a soluble polypeptide, followed by one or more concentration, salting-out, ion exchange, affinity purification or size exclusion chromatography steps. Microbial cells can be disrupted by any convenient method, including freeze-thaw cycling, sonication, mechanical disruption, or use of cell lysing agents.

In another embodiment, the invention provides a process for producing cells capable of expressing a polypeptide according to the invention, comprising genetically engineering cells with a vector or a nucleic acid according to the invention

In another embodiment, the invention provides a process for producing antibodies or fragments thereof according to the invention comprises culturing a host cell transformed with an expression vector comprising a nucleic sequence that encodes said antibodies or fragments thereof under conditions sufficient to promote expression of said polypeptides. The antibody or fragment thereof according to the invention is then recovered from culture medium or cell extracts, depending upon the expression system employed. As known to the skilled artisan, procedures for purifying a recombinant protein will vary according to such factors as the type of host cells employed and whether or not the recombinant protein is secreted into the culture medium as described above.

### Compositions

The invention provides pharmaceutical or therapeutic agents as compositions and methods for treating a patient, preferably a mammalian patient, and most preferably a human patient who is suffering from a medical disorder, and in particular an inflammatory and/or autoimmune disease or a cancer. Alternatively, the invention provides methods for preventing a medical disorder, and in particular an inflammatory and/or autoimmune disease or a cancer.

In one embodiment, is provided a pharmaceutical composition comprising one or more of (i) an antibody specific for MMP9 or antigen-binding fragment thereof according to the invention, (ii) a nucleic acid according to the invention, (iii) a vector according to the invention, and/or (iv) a host cell according to the invention, and at least one pharmaceutically acceptable carrier.

Pharmaceutical compositions of the invention can contain one or more antibodies specific for MMP9 or antigen-binding fragment thereof in any form described herein. Compositions of this invention may further comprise one or more pharmaceutically acceptable additional ingredient(s) such as alum, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as tablets or filled capsules, freeze-dried forms, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions of this invention may be liquid formulations including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agent include, but are not limited to, sorbitol syrup, methylcellulose, glucose/sugar syrup, gelatin, hydroxyethylcellulose, carboxymethylcellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in Part 5 of Remington's The Science and Practice of Pharmacy, 22nd Edition, 2012, Pharmaceutical Press and the University of the Sciences, Philadelphia College of Pharmacy*,* which is incorporated herein by reference.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate.

Tablets may be coated according to methods well known in the art.

Injectable compositions are typically based upon injectable sterile saline or phosphate-buffered saline or other injectable carriers known in the art.

Compositions of this invention may also be formulated as transdermal formulations comprising aqueous or non-aqueous vehicles including, but not limited to, creams, ointments, lotions, pastes, medicated plaster, patch, or membrane.

Compositions of this invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

The compounds of this invention can also be administered in sustained release forms or from sustained release drug delivery systems. A description of representative sustained release materials can also be found in the incorporated materials in *Remington's*

### Pharmaceutical Sciences.

Injectable formulations are particularly appropriate for administering the compositions according to the invention.

In another embodiment, the invention provides an imaging composition or diagnostic composition comprising an antibody specific for MMP9 or antigen-binding fragment thereof as described herewith.

The imaging composition or diagnostic composition according to the invention is useful for detecting elevated levels of MMP9 associated with inflammatory and/or autoimmune diseases or cancers.

### Combination

According to the invention, an antibody specific for MMP9 or antigen-binding fragment thereof according to the invention can be administered alone or in combination with a co-agent useful in the prevention and/or treatment of an inflammatory and/or autoimmune disease, for example immune modulatory drugs including biologics, small molecules, and vaccines.

Alternatively, an antibody specific for MMP9 or antigen-binding fragment thereof according to the invention can be administered or in combination with a co-agent useful in the treatment of cancer, for example an anti-cancerous drug such as cytotoxic drugs, tyrosine kinase inhibitors imatinib (Gleevec/Glivec) or gefitinib (Iressa), and therapeutic antibodies such as trastuzumab (Herceptin) or anti-CD20 antibody rituximab.

The invention encompasses the administration of an antibody specific for MMP9 or antigen-binding fragment thereof wherein the antibody or fragment thereof is administered to an individual prior to, simultaneously or sequentially with other therapeutic regimens or co-agents useful in the prevention and /or treatment of an inflammatory and/or autoimmune disease or cancer, in a therapeutically effective amount. The antibody specific for MMP9 or antigen-binding fragment thereof according to the invention that are administered simultaneously with said co-agents can be administered in the same or different compositions and in the same or different routes of administration.

In a particular embodiment, an antibody specific for MMP9 or antigen-binding fragment thereof according to the invention can be administered in combination with an anti-TNFα antibody for the treatment of subjects suffering from moderate to severe Crohn's disease.

### Mode of administration

Compositions of this invention may be administered in any manner including, but not limited to, orally, parenterally, sublingually, transdermally, rectally, transmucosally, topically, via inhalation, via buccal or intranasal administration or intra bladder, or combinations thereof.

Parenteral administration includes, but is not limited to, intravenous, intra-arterial, intraperitoneal, subcutaneous, intramuscular, intra-thecal, and intra-articular. The compositions of this invention may also be administered in the form of an implant, which allows slow release of the compositions as well as a slow controlled i.v. infusion. In a particular embodiment, an antibody specific for MMP9 or antigen-binding fragment thereof according to the invention is administered systemically or locally.

In a particular embodiment, an antibody specific for MMP9 or antigen-binding fragment thereof according to the invention is administered by subcutaneous or intravenous route.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

Typically, therapeutically effective amounts of a pharmaceutically active antibody range from 1 mg to up to 50 mg/kg body weight dose. If the regimen is a continuous infusion, it may be in the range of 0.250 mg to 13 mg per kg of body weight.

### Patients

Typically, patients according to the invention are patients suffering from a MMP9-related disease, in particular a MMP9-related disease selected from the group consisting of inflammatory and autoimmune diseases, cancers or tumors, lung diseases, fibrotic lung diseases, septicemia, muscular dystrophy, allergy, renal fibrosis, scleroderma, dilated cardiomyopathy, Chagas disease, cardiovascular diseases, neuropsychiatric disorders, diabetes, and eye diseases, or any other MMP9-related disease or disorder.

In an embodiment, patients according to the invention are patients suffering from an inflammatory and/or autoimmune disease including, for instance, inflammatory bowel diseases (IBD) including Crohn's disease (CD), ulcerative colitis (UC), indeterminate colitis, collagenous colitis, rheumatoid arthriris (RA), multiple sclerosis (MS), systemic lupus erythematosus (SLE), Sjogren's syndrome, systemic sclerosis, polymyositis, atherosclerosis.

In a particular embodiment, patients according to the invention are patients suffering from inflammatory bowel disease, more particularly penetrating and stricturing Crohn's disease and ulcerative colitis.

In another embodiment, patients according to the invention are patients suffering from a lung disease including asthma, fibrotic lung diseases such as idiopathic pulmonary, chronic obstructive pulmonary disease (COPD), or a disease selected from septicemia, muscular dystrophy, allergy, renal fibrosis, scleroderma, dilated cardiomyopathy, Chagas disease, cardiovascular diseases, neuropsychiatric disorders, diabetes, and eye diseases.

In another embodiment, patients according to the invention are patients suffering from a cancer or tumor including, for instance, haematopoetic cancer, brain cancer, breast cancer, colorectal cancer, head and neck cancer, pancreatic cancer, lung cancer, liver cancer, melanoma, prostate cancer, muscle cancer, mesenchymal cancer, esophagogastric adenocarcinoma, non-small lung cancer, lung squamous cell carcinoma, lung adenocarcinoma, gastric adenocarcinoma, pancreatic adenocarcinoma, hepathocellular carcinomacolorectal cancer and hepathocellular carcinoma.

In a particular embodiment, patients according to the invention are patients suffering from colorectal cancer or adenocarcinoma.

### Uses and methods according to the invention

The antibody specific for MMP9 or antigen-binding fragment thereof, the nucleic acids, the vectors, the host cells, the compositions according to the invention are for use in the diagnosis, prevention or treatment of disorders associated with, caused by, or accompanied by elevated levels of MMP9 and/or elevated MMP9 activity.

In one embodiment is provided an antibody specific for MMP9 or antigen-binding fragment thereof according to the invention for use as a medicament.

Another embodiment provides an antibody or fragment thereof according to the invention for use in the prevention and/or treatment of a MMP9-related disease selected from the group consisting of inflammatory and autoimmune diseases, cancers or tumors, lung diseases, fibrotic lung diseases, septicemia, muscular dystrophy, allergy, renal fibrosis, scleroderma, dilated cardiomyopathy, Chagas disease, cardiovascular diseases, neuropsychiatric disorders, diabetes, and eye diseases, or any other MMP9-related disease or disorder.

Another particular embodiment provides an antibody or fragment thereof according to the invention for use in the prevention and/or treatment of an inflammatory and/or autoimmune disease, in particular inflammatory bowel diseases (IBD) including Crohn's disease (CD), ulcerative colitis (UC), indeterminate colitis, collagenous colitis, rheumatoid arthriris (RA), multiple sclerosis (MS), systemic lupus erythematosus (SLE), Sjogren's syndrome, systemic sclerosis, polymyositis, atherosclerosis.

Another particular embodiment provides an antibody or fragment thereof according to the invention for use in the prevention and/or treatment of a lung disease including asthma, fibrotic lung diseases such as idiopathic pulmonary, chronic obstructive pulmonary disease (COPD), or a disease selected from septicemia, muscular dystrophy, allergy, renal fibrosis, scleroderma, dilated cardiomyopathy, Chagas disease, cardiovascular diseases, neuropsychiatric disorders, diabetes, and eye diseases.

A still other particular embodiment provides an antibody or fragment thereof according to the invention for use in the prevention and/or treatment of a cancer or tumor, in particular a cancer selected from the group consisting of haematopoetic cancer, brain cancer, breast cancer, colorectal cancer, head and neck cancer, pancreatic cancer, lung cancer, liver cancer, melanoma, prostate cancer, muscle cancer, mesenchymal cancer, esophagogastric adenocarcinoma, non-small lung cancer, lung squamous cell carcinoma, lung adenocarcinoma, gastric adenocarcinoma, pancreatic adenocarcinoma, hepathocellular carcinomacolorectal cancer and hepathocellular carcinoma, more particularly colorectal cancer or adenocarcinoma.

In another embodiment is provided a use of an antibody specific for MMP9 or antigen-binding fragment thereof according to the invention for the preparation of a pharmaceutical composition for preventing and/or treating a MMP9-related disease selected from the group consisting of inflammatory and autoimmune diseases, cancers or tumors, lung diseases, fibrotic lung diseases, septicemia, muscular dystrophy, allergy, renal fibrosis, scleroderma, dilated cardiomyopathy, Chagas disease, cardiovascular diseases, neuropsychiatric disorders, diabetes, and eye diseases, or any other MMP9-related disease or disorder.

In one particular embodiment is provided a use of an antibody specific for MMP9 or antigen-binding fragment thereof according to the invention for the preparation of a pharmaceutical composition for preventing and/or treating an inflammatory and/or autoimmune disease in a subject, in particular for preventing or treating inflammatory bowel diseases (IBD) including Crohn's disease (CD), ulcerative colitis (UC), indeterminate colitis, collagenous colitis, rheumatoid arthriris (RA), multiple sclerosis (MS), systemic lupus erythematosus (SLE), Sjogren's syndrome, systemic sclerosis, polymyositis, atherosclerosis.

In a specific embodiment is provided a use of an antibody specific for MMP9 or antigen-binding fragment thereof according to the invention for the preparation of a pharmaceutical composition for preventing and/or treating inflammatory bowel disease, in particular penetrating and stricturing Crohn's disease and ulcerative colitis.

In one embodiment is provided a use of an antibody specific for MMP9 or antigen-binding fragment thereof according to the invention for the preparation of a pharmaceutical composition for preventing and/or treating a lung disease including asthma, fibrotic lung diseases such as idiopathic pulmonary, chronic obstructive pulmonary disease (COPD), or a disease selected from septicemia, muscular dystrophy, allergy, renal fibrosis, scleroderma, dilated cardiomyopathy, Chagas disease, cardiovascular diseases, neuropsychiatric disorders, diabetes, and eye diseases.

In an alternative embodiment is provided a use of an antibody specific for MMP9 or antigen-binding fragment thereof according to the invention for the preparation of a pharmaceutical composition for preventing and/or treating a cancer or tumor, in particular for preventing and/or treating a cancer selected from the group consisting of haematopoetic cancer, brain cancer, breast cancer, colorectal cancer, head and neck cancer, pancreatic cancer, lung cancer, liver cancer, melanoma, prostate cancer, muscle cancer, mesenchymal cancer, esophagogastric adenocarcinoma, non-small lung cancer, lung squamous cell carcinoma, lung adenocarcinoma, gastric adenocarcinoma, pancreatic adenocarcinoma, hepathocellular carcinomacolorectal cancer and hepathocellular carcinoma.

In a specific embodiment is provided a use of an antibody specific for MMP9 or antigen-binding fragment thereof according to the invention for the preparation of a pharmaceutical composition for preventing and/or treating colorectal cancer or adenocarcinoma.

In another embodiment is provided a method for preventing and/or treating a MMP9-related disease selected from the group consisting of inflammatory and autoimmune diseases, cancers or tumors, lung diseases, fibrotic lung diseases, septicemia, muscular dystrophy, allergy, renal fibrosis, scleroderma, dilated cardiomyopathy, Chagas disease, cardiovascular diseases, neuropsychiatric disorders, diabetes, and eye diseases, or any other MMP9-related disease or disorder comprising administering a therapeutically effective amount of an antibody specific for MMP9 or antigen-binding fragment thereof according to the invention, to a subject in need thereof.

In a particular embodiment is provided a method for preventing and/or treating an inflammatory and/or autoimmune disease, in particular for preventing or treating inflammatory bowel diseases (IBD) including Crohn's disease (CD), ulcerative colitis (UC), indeterminate colitis, collagenous colitis, rheumatoid arthriris (RA), multiple sclerosis (MS), systemic lupus erythematosus (SLE), Sjogren's syndrome, systemic sclerosis, polymyositis, atherosclerosis, comprising administering a therapeutically effective amount of an antibody specific for MMP9 or antigen-binding fragment thereof according to the invention, to a subject in need thereof.

In a specific embodiment is provided a method of preventing and/or treating an inflammatory bowel disease, in particular for preventing or treating penetrating and stricturing Crohn's disease and ulcerative colitis, comprising administering a therapeutically effective amount of an antibody specific for MMP9 or antigen-binding fragment thereof according to the invention, to a subject in need thereof.

In another embodiment is provided a method for preventing and/or treating a lung disease including asthma, fibrotic lung diseases such as idiopathic pulmonary, chronic obstructive pulmonary disease (COPD), or a disease selected from septicemia, muscular dystrophy, allergy, renal fibrosis, scleroderma, dilated cardiomyopathy, Chagas disease, cardiovascular diseases, neuropsychiatric disorders, diabetes, and eye diseases, comprising administering a therapeutically effective amount of an antibody specific for MMP9 or antigen-binding fragment thereof according to the invention, to a subject in need thereof.

In an alternative embodiment is provided a method of preventing and/or treating a cancer or tumor, in particular for preventing and/or treating a cancer selected from the group consisting of haematopoetic cancer, brain cancer, breast cancer, colorectal cancer, head and neck cancer, pancreatic cancer, lung cancer, liver cancer, melanoma, prostate cancer, muscle cancer, mesenchymal cancer, esophagogastric adenocarcinoma, non-small lung cancer, lung squamous cell carcinoma, lung adenocarcinoma, gastric adenocarcinoma, pancreatic adenocarcinoma, hepathocellular carcinomacolorectal cancer and hepathocellular carcinoma, comprising administering a therapeutically effective amount of an antibody specific for MMP9 or antigen-binding fragment thereof according to the invention, to a subject in need thereof.

In a specific embodiment is provided a method for preventing and/or treating colorectal cancer or adenocarcinoma comprising administering a therapeutically effective amount of an antibody specific for MMP9 or antigen-binding fragment thereof according to the invention, to a subject in need thereof.

In an alternative embodiment is provided a method of detecting MMP9 in a biological sample comprising contacting a biological sample from a subject with an antibody specific for MMP9 or antigen-binding fragment thereof according to the invention.

As used herewith "biological sample" refers to cells, tissue samples or cell components (such as cellular membranes or cellular components) obtained from a subject, in particular from a subject suspected of, or suffering from, inflammatory or autoimmune disease and/or cancer or tumor or at high risk of developing such a disorder.

Examples of biological sample include blood, serum, plasma, cerebrospinal fluid, synovial fluid, and tissue samples including cells isolated from said tissue. Tissue samples include formalin-fixed or frozen tissue sections.

Any suitable method for detection and analysis of MMP9 can be employed, including diagnostic assay techniques known in the art such as competitive binding assays, direct or indirect sandwich assays and immunoprecipitation assays conducted in either heterogeneous or homogeneous phases.

In a particular embodiment, the invention provides an *ex vivo* method for detecting the presence and/or concentration of MMP9 protein in a biological sample, comprising the steps of:
(i) Providing a biological sample from a subject,
(ii) Reacting said biological sample with at least one antibody or antigen-binding fragment thereof according to the invention, under conditions sufficient for binding MMP9 protein present in said biological sample to said at least one antibody or fragment thereof through antigen-antibody interactions; and
(iii) Detecting a signal proportional to the level of antigen-antibody complex formed in step (ii),
wherein the intensity of the signal correlates with the concentration of MMP9 protein in the biological sample.

More particularly, it is provided an *ex-vivo* method of prognosis or diagnosis of an inflammatory and/or autoimmune disease or a cancer associated with an elevated level of MMP9 from a biological sample of a subject comprising the steps of:
(a) Providing a biological sample from a subject;
(b) Bringing said biological sample into contact with a solid matrix where at least one antibody or fragment thereof according to the invention is bound to, wherein the contacting is under conditions sufficient for binding MMP9 protein present in said biological fluid sample to said at least one antibody or fragment thereof through antigen-antibody interactions;
(c) Removing any unbound MMP9 protein from the surface of said solid matrix;
(d) Detecting a signal proportional to the level of antigen-antibody complex bound to said solid matrix,
(e) Comparing the level of signal detected in step (d) with the level of signal detected in the same conditions with a negative control,
wherein a level of signal detected in the subject's sample that is higher than the level of signal detected in the negative control is indicative of an elevated level of MMP9 associated with an inflammatory and/or autoimmune disease or a cancer.

### Kit

One aspect of the invention relates to a kit comprising at least one antibody or antigen-binding fragment thereof according to the invention, and/or at least one nucleic acid encoding said antibody or fragment thereof, and/or at least one vector comprising said nucleic acid, and/or at least one host cell according to the invention, and optionally instructional material.

In a particular embodiment, the kit according to the invention comprises at least one antibody or antigen-binding fragment thereof according to the invention, to be coupled or already coupled to a solid matrix.

Examples of solid matrix suitable for the invention include any solid phase support suitable for carrying out an immunoassay or a method according to the invention, such as beads, microparticles, nanoparticles, tubes, fabrics or plates, films, slides, wells, formed from or coated with glass, polystyrene, polypropylene, nitrocellulose, quartz, ceramic, dextran or other materials. For example, the solid matrix is in a form of microtiter wells, such as a 96-well microtiter plate.

The fixation of the antibodies or fragments thereof according to the invention to the solid matrix in a kit according to the invention may be carried out by adsorption or chemical coupling to a solid phase support. Any means known in the art for immobilizing a protein or peptide to a solid support can be used. The antibodies or fragments thereof according to the invention can be either covalently or non-covalently bound to the solid matrix by techniques such as covalent bonding via an amide or ester linkage or adsorption. Peptides can be bound using binding pairs such as biotin and avidin or antibody and antigen. After the peptides are affixed to the solid matrix, the solid matrix can be incubated with a blocking solution (containing a blocking protein such as bovine serum albumin) to reduce non-specific adsorption of antibodies in a test sample to the support surface. According to one aspect, the antibodies or fragment thereof according to the invention can be synthesized directly on the solid matrix of the kit of the invention.

According to one embodiment, when the kit comprises at least one antibody or fragment thereof according to the invention or a combination thereof for coupling to a solid matrix as solid phase support, the kit further optionally comprises coupling reagents and/or a solid matrix for performing an immunoassay.

According to another further embodiment, the kit according to the invention further comprises at least one rinsing reagent for washing unbound material before detection in order to avoid background noise detection. Typically rinsing reagents comprise standard buffers known in the art.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### EXAMPLES

### Example 1: Generation and isolation of the anti-MMP9 antibodies according to the invention

The anti-MMP9 antibodies according to the invention were obtained by carrying out the following steps:

### 1/ Phage display Hit Discovery (HD)

A ScFv library was used as a source of ScFv fragments. This library is naive in origin (i.e. constructed using PBMCs of non-immunised healthy human donors), of modest size (about 2.5.10⁹ VH/VL combinations) and contains VH content derived from the clonally unselected and non-class-switched IgM repertoire. Using this library, ScFv candidates were first selected based on their binding to human or mouse MMP9 (full-length, pro-, and activated forms) by phage display, the ScFvs were reformatted into IgG1 and screened for functional neutralization of MMP9 activity (in particular by determining IC₅₀, species selectivity, modality of neutralization (proMMP9 or MMP9)). This step allowed the identification of 10 candidate antibodies, which could be divided into two mechanistic classes: those blocking MMP9 activity by interfering with the activation of latent proMMP9 (suggesting that the pro-domain is part of the epitope recognized by the antibodies) and those interfering directly with the catalytic activity of activated MMP9 (suggesting that the CAT_fn domain is part of the epitope recognized by the antibodies).

### 2/ Hit Optimization (HO) by VL chain shuffling for affinity maturation:

Four of the HD candidates obtained in the previous step were subjected to hit optimization via lambda light chain shuffling to improve their potency. Basically, parental HD clone VH chains were permutated against the entire VL lambda content of the original library, generating several new clone-specific libraries.

### 3/ Lead optimization:

A HO candidate (G03-VH/G06-VL) was selected for lead optimization and characterization, through in particular (i) optimization by germlining framework residues (as defined by Kabat) to minimize sequence deviation from closest human germline to potentially improve biophysical properties and reduce immunogenicity risk and (ii) change some amino acids or amino acid motifs within the CDRs which might lead to chemical instability or aggregation of antibodies. This strategy allowed the generation of antibodies comprising the VH/VL regions indicated in above Table 2.

In the following examples, the specific VH/VL regions mentioned in Table 3 that follows were reformatted as human IgG4 for further characterization. For this purpose, the nucleic acids encoding the VH and VL chains of interest were cloned in pTT vectors (engineered for cloning compatibility with original chains) for transient co-expression of IgG4 in HEK293 cells.

**Table 3**

| **Name** | **Sequence of Heavy chain variable region** | **Sequence of Light chain variable region** |
|---|---|---|
| G03-VH-GL/ G06-VL-GL1 | SEQ ID NO: 9 | SEQ ID NO: 17 |
| G03-VH-GL-V1 / G06-VL-GL1 | SEQ ID NO: 10 | SEQ ID NO: 17 |
| G03-VH-GL1-V1-V9/ G06-VL-GL1 | SEQ ID NO: 12 | SEQ ID NO: 17 |

### Example 2: Potency of some anti-MMP9 antibodies according to the invention in human MMP3/MMP9 and human MMP9 activity assays

The functional neutralization of MMP9 catalytic activity towards the fluorogenic polymer substrate DQ-gelatin by some antibodies according to the invention was evaluated.

The signature activity of MMP9 (and MMP2) is gelatin degradation. Gelatin is essentially an irreversibly denatured form of various collagens, several of which are considered key physiological substrates for MMP9. Unlike small peptidic substrates, the binding and recognition of gelatin (collagen) by MMP9 is complex and is known to be mediated via regions of the molecule other than the active site, including the fibronectin and PEX domains. In order to retain these physiologically relevant 'exosite' interactions and to facilitate the isolation of potential 'non-classical' allosteric neutralizer classes, a fluorogenically quenched soluble gelatin polymer (DQ-gelatin) was chosen as the key substrate for plate-based screening and ranking purposes. Substrate hydrolysis was mediated by full length or truncated fragments of activated MMP9 (ie. pro-domain removed). The assay could be performed in two modes.

The first of these utilized pre-activated MMP9 (engineered to contain an LETD caspase 8 recognition motif at the native cleavage junction) in which the pro-domain was first removed via directed caspase 8 cleavage ("MMP9 assay"). The second assay ("MMP3/MMP9 assay") mode was devised to incorporate the removal of the pro-domain (i.e. the activation step) as an additional linked process within the assay. This was achieved by combining catalytic domain of human MMP3 and full length (pro-form, inactive) MMP9 in the assay cocktail together with the DQ-gelatin substrate and test antibody. MMP3 is considered a strong candidate for a physiologically relevant activator of MMP9 and has been shown to remove the pro-domain via a two-step sequential mechanism (Ogata et al, 1992, J. Biol. Chem. 267(6): 3581-4*)* and is frequently co-expressed with MMP9 in disease tissues. Importantly, activated MMP3 itself does not appear to significantly cleave the DQ-gelatin substrate. Hence, DQ-gelatin hydrolysis (emission of fluorescence) in this assay is dependent on MMP9 activation and MMP9 catalytic activity, which offers the potential to detect neutralizers that interfere with either process.

Thus, the "MMP3/MMP9 assay" was used to determine the ability of anti-MMP-9 antibodies to neutralize proMMP9 activation and its downstream catalytic activity. Briefly, aliquots of recombinant human proMMP9 were pre-incubated with various dilutions of test antibodies for one hour. The digestion was started by adding a recombinant catalytic domain of human MMP-3, together with the MMP-9-specific fluorescent substrate DQ-Gelatin. Emitted fluorescence signal is proportional to the digestion of the gelatin substrate, thus to the catalytic activity of mature MMP-9 enzyme. The fluorescence signal was plotted against the antibody concentrations and half-maximal inhibitory potencies (IC₅₀ values) are deduced from non-linear regression curves.

In addition, the "MMP9" assay was used to determine the capacity of anti-MMP-9 antibodies to directly neutralize the catalytic activity of mature MMP9. Briefly, the assay is similar to the MMP3/MMP9 assay but uses a catalytically active caspase-cleaved recombinant MMP-9 and the addition of recombinant catalytic domain of human MMP-3 is not needed.

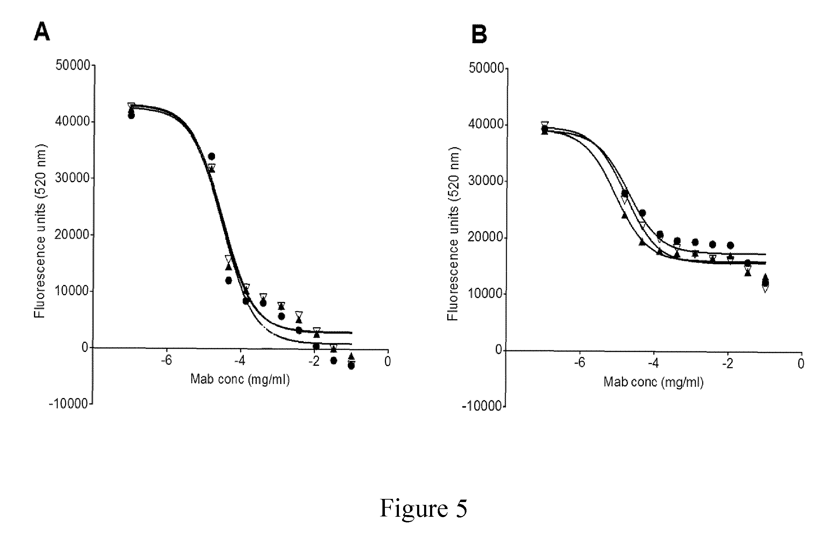

All antibodies according to the invention, which were tested, were able to effectively block both processing of the preproenzyme or proenzyme to the catalytically active enzyme and MMP9 catalytic activity (Table 4, Figure 5). They appeared to block the sites of proteolytic cleavage in MMP9 and the enzymatic activity mainly by interfering with the propeptide and the catalytic site of activated MMP9 directly.

**Table 4. Potency to neutralize processing of human proMMP9 and/or its downstream catalytic activity on gelatin.**

| **Variable region (heavy/light variable regions) comprised in the antibody** | **Human MMP9 (i.e. activated MMP9)** | | **Human proMMP9** | |
|---|---|---|---|---|
| | **IC₅₀ (nM)** | **Efficiency** | **IC₅₀ (nM)** | **Efficiency** |
| G03-VH-GL / G06-VL-GL1 | 0.13 | 50% | 0.21 | 100% |
| G03-VH-GL-V1/ G06-VL-GL1 | 0.06 | 50% | 0.19 | 100% |
| G03-VH-GL-V1-V9 / G06-VL-GL1 | 0.11 | 50% | 0.21 | 100% |

### Example 3: Species-specificity of some anti-MMP9 antibodies according to the invention in MMP3/MMP9 assay

The species-specificity of the enzymatic inhibitory activities of anti-MMP9 antibodies was evaluated using the "MMP3/MMP9 assay" described in the Example 2. The enzyme used was either Cynomologus monkey (cyno), rat or mouse proMMP9.

All antibodies according to the invention, which were tested, were able to efficiently block the activation and/or the downstream catalytic activity of Cynomologus monkey (cyno) and rat proMMP9 (Tables 5 and 6, respectively), and to mouse proMMP9 (Table 7).

**Table 5. Potency to neutralize processing of cyno MMP9 and/or its catalytic activity on gelatin.**

| **Variable region (heavy/light variable regions) comprised in the antibody** | **Cyno proMMP9** | |
|---|---|---|
| | **IC₅₀ (nM)** | **Efficiency** |
| G03-VH-GL / G06-VL-GL1 | 0.04 | 100% |
| G03-VH-GL-V1/ G06-VL-GL1 | 0.03 | 100% |
| G03-VH-GL-V1-V9 / G06-VL-GL1 | 0.14 | 100% |

**Table 6. Potency to neutralize processing of rat MMP9 and/or its catalytic activity on gelatin.**

| **Variable region (heavy/light variable regions) comprised in the antibody** | **rat proMMP9** | |
|---|---|---|
| | **IC₅₀ (nM)** | **Efficiency** |
| G03-VH-GL / G06-VL-GL1 | 64.67 | 100% |
| G03-VH-GL-V1/ G06-VL-GL1 | 18.96 | 100% |
| G03-VH-GL-V1-V9 / G06-VL-GL1 | 93.62 | 100% |

**Table 7. Potency to neutralize processing of mouse MMP9 and/or its catalytic activity on gelatin.**

| **Variable region (heavy/light variable regions) comprised in the antibody** | **mouse proMMP9** | |
|---|---|---|
| | **IC₅₀ (nM)** | **Efficiency** |
| G03-VH-GL / G06-VL-GL1 | 151 | 30% |
| G03-VH-GL-V1/ G06-VL-GL1 | 16.38 | 25% |
| G03-VH-GL-V1-V9 / G06-VL-GL1 | 12.58 | 25% |

### Example 4: MMP selectivity of some anti-MMP9 antibodies according to the invention in human MMPs activity assay

The selectivity of the neutralization of MMP9 catalytic activity towards a fluorogenic peptide substrate by some antibodies according to the invention was evaluated.

The selectivity of anti-MMP9 antibodies was evaluated in an assay assessing MMPs mediated cleavage of a fluorogenic peptide substrate, OmniMMP^{™} RED (Enzo - BML-P277-9090), using the MMP Inhibitor Profiling Kit (Enzo - BML-AK308). Briefly, aliquots of recombinant catalytic domain of various human MMPs (MMP1, MMP2, MMP3, MMP8, MMP9, MMP12, MMP13, MMP14 and MMP19) were pre-incubated with a fixed concentration (100 nM) of test antibodies for one hour. The MMP inhibitor NNGH (Enzo - BML-PI115-9090) and anti-MMP2/9 (539A-M0237-D02), anti-MMP9 (539A-M0240-B03) and isotype control (anti-HEL IgG1) antibodies were included in the test, as positive and negative controls. Fluorogenic peptide substrate was then added and fluorescence was measured. Emitted fluorescence signal is proportional to the digestion of the peptidic substrate, thus to the activity of MMP catalytic domains. The fluorescence signal in the absence of inhibitor was normalized to 100% activity. Signal obtained in the presence of each inhibitor was given relative to this value and referred as to percentage of remaining activity.

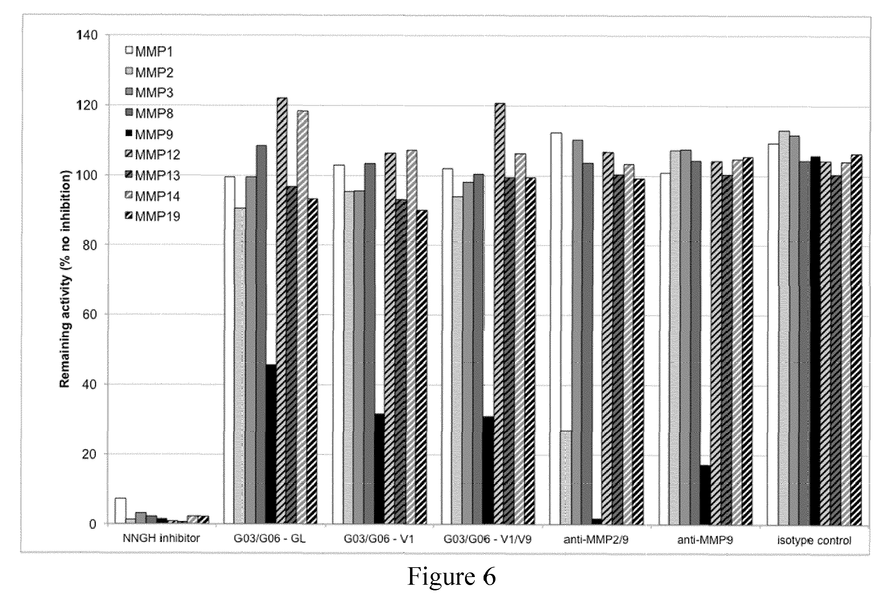

All antibodies according to the invention, which were tested, were able to efficiently block the catalytic activity of human MMP9, while they had no significant effect on the catalytic activity of the eight other human MMPs tested (Figure 6).

### LIST OF SEQUENCES

**Amino acid sequence of Human MMP9** (NCBI Reference Sequence: NP_004985.2)
SEQ ID NO: 1

**Amino acid sequence of G03-H-CDR1**
SEQ ID NO: 2: SYGIS

**Amino acid sequence of G03-H-CDR2**
SEQ ID NO: 3: WISAYNGNTNYAQKLQG

**Amino acid sequence of G03-H-CDR3**
SEQ ID NO: 4: DYYGMDV

**Amino acid sequence of G03-H-CDR2-V1**
SEQ ID NO: 5: **Y**ISAYNGNTNYAQKLQG

**Amino acid sequence of G03-H-CDR2-V9**
SEQ ID NO: 6: WISAYN**A**NTNYAQKLQG

**Amino acid sequence of G03-H-CDR2-V1-V9**
SEQ ID NO: 7: **Y**ISAYN**A**NTNYAQKLQG

**Amino acid sequence of G03-VH**
SEQ ID NO: 8:

QVQLVESGAEVKKPGASVKVSCKASGYTFTSYGISWVRQAPGQGLEWMGWISAYNGNT
NYAQKLQGRVTMTTDTSTNTAHMELRSLRSDDTAVYYCARDYYGMDVWGQGTTVTVSS

**Amino acid sequence of G03-VH-GL**
SEQ ID NO: 9:

EVQLVQSGAEVKKPGASVKVSCKASGYTFTSYGISWVRQAPGQGLEWMGWISAYNGNT
NYAQKLQGRVTMTTDTSTSTAYMELRSLRSDDTAVYYCARDYYGMDVWGQGTTVTVSS

**Amino acid sequence of G03-VH-GL-V1**
**SEQ ID NO: 10:**

EVQLVQSGAEVKKPGASVKVSCKASGYTFTSYGISWVRQAPGQGLEWMGYISAYNGNT
NYAQKLQGRVTMTTDTSTSTAYMELRSLRSDDTAVYYCARDYYGMDVWGQGTTVTVSS

**Amino acid sequence of G03-VH-GL-V9**
SEQ ID NO: 11:

EVQLVQSGAEVKKPGASVKVSCKASGYTFTSYGISWVRQAPGQGLEWMGWISAYNANT
NYAQKLQGRVTMTTDTSTSTAYMELRSLRSDDTAVYYCARDYYGMDVWGQGTTVTVSS

**Amino acid sequence of G03-VH-GL-V1-V9**
SEQ ID NO: 12:

EVQLVQSGAEVKKPGASVKVSCKASGYTFTSYGISWVRQAPGQGLEWMGYISAYNANT
NYAQKLQGRVTMTTDTSTSTAYMELRSLRSDDTAVYYCARDYYGMDVWGQGTTVTVSS

**Amino acid sequence of G06-L-CDR1**
SEQ ID NO: 13: SGSTFNIGSNTVN

**Amino acid sequence of G06-L-CDR2**
SEQ ID NO: 14: SDSHRPS

**Amino acid sequence of G06-L-CDR3**
SEQ ID NO: 15: QAWDSSAVV

**Amino acid sequence of G06-VL**
SEQ ID NO: 16:

QAVLTQPPSTSGTPGQRVTISCSGSTFNIGSNTVNWYQQLPGTAPKLLIYSDSHRPSG
VPDRFSGSNSWNTATLTISRVEVGDEGDYYCQAWDSSAVVFGGGTKVTVLG

**Amino acid sequence of G06-VL-GL1**
SEQ **ID** NO: **17:**

QAVLTQPPSASGTPGQRVTISCSGSTFNIGSNTVNWYQQLPGTAPKLLIYSDSHRPSG
VPDRFSGSNSWNTASLAISGLQSEDEADYYCQAWDSSAVVFGGGTKVTVLG

**Nucleic acid sequence of G03-H-CDR1**
SEQ ID NO: 18: agttacggcatctct

**Nucleic acid sequence of G03-H-CDR2**
SEQ ID NO: 19:
tggatctccgcctataatggtaatacaaactatgcgcagaaactccaaggc

**Nucleic acid sequence of G03-H-CDR3**
SEQ ID NO: 20: gactattacggcatggacgtg

**Nucleic acid sequence of G03-H-CDR2-V1**
SEQ ID NO: 21:
**tat**attagcgcttacaacggaaacaccaattacgcccagaagctccagggc

**Nucleic acid sequence of G03-H-CDR2-V1-V9**
SEQ ID NO: 22**:**
**tat**attagtgcatataac**gca**aataccaattacgctcaaaaactccaagga

**Nucleic acid sequence of G06-L-CDR1**
SEQ ID NO: 23: tcgggttctacatttaatataggaagtaatactgtgaat

**Nucleic acid sequence of G06-L-CDR2**
SEQ ID NO: 24: agcgacagtcacagaccatct

**Nucleic acid sequence of G06-L-CDR3**
SEQ ID NO: 25: caagcctgggacagctcagctgtcgta

**Amino acid sequence of engineered human IgG4 heavy chain constant region**
SEQ ID NO: 26:

ASTKGPSVFPLAPCSRSTSESTAALGCLVKDYFPEPVTVSWNSGALTSGVHTFPAVLQ
SSGLYSLSSVVTVPSSSLGTKTYTCNVDHKPSNTKVDKRVESKYGPPCPPCPAPEFLG
GPSVFLFPPKPKDTLMISRTPEVTCVVVDVSQEDPEVQFNWYVDGVEVHNAKTKPREE
QFNSTYRVVSVLTVLHQDWLNGKEYKCKVSNKGLPSSIEKTISKAKGQPREPQVYTLP
PSQEEMTKNQVSLTCLVKGFYPSDIAVEWESNGQPENNYKTTPPVLDSDGSFFLYSKL
TVDKSRWQEGNVFSCSVMHEALHNHYTQKSLSLSLG

## Claims

1. An isolated antibody specific for MMP9 or antigen-binding fragment thereof comprising a heavy chain variable region comprising:
(i) a heavy chain CDR1 of SEQ ID NO: 2 or a variant thereof wherein 1, 2, or 3 amino acids of said heavy chain CDR1 is substituted by a different amino acid;
(ii) a heavy chain CDR2 of SEQ ID NO: 3 or a variant thereof wherein 1, 2, or 3 amino acids of said heavy chain CDR2 is substituted by a different amino acid;
(iii) a heavy chain CDR3 of SEQ ID NO: 4 or a variant thereof wherein 1, 2, or 3 amino acids of said heavy chain CDR3 is substituted by a different amino acid.

2. The isolated antibody or fragment thereof according to claim 1, wherein the heavy chain variable region comprises SEQ ID NO: 9 or SEQ ID NO: 8, or a variant thereof wherein 1, 2, or 3 amino acids of at least one of the heavy chain CDR1, CDR2, and/or CDR3, and/or 1, 2, 3, 4, 5, 6, 7, 8, 9, 10, 11, 12, 13, 14, or 15 amino acids of the heavy chain variable framework region is substituted by a different amino acid.

3. The isolated antibody or fragment thereof according to any one of claims 1 to 2, wherein said heavy chain variable region comprises an amino acid sequence selected from:
(i) the amino acid sequence SEQ ID NO: 10,
(ii) the amino acid sequence SEQ ID NO: 12,
(iii) the amino acid sequence SEQ ID NO: 9,
(iv) the amino acid sequence SEQ ID NO: 11,
(v) the amino acid sequence of SEQ ID NO: 8.

4. The isolated antibody or fragment thereof according to any one of claims 1 to 3, further comprising a light chain variable region comprising:
(i) a light chain CDR1 of SEQ ID NO: 13 or a variant thereof wherein 1, 2, or 3 amino acids of said light chain CDR1 is substituted by a different amino acid;
(ii) a light chain CDR2 of SEQ ID NO: 14 or a variant thereof wherein 1, 2, or 3 amino acids of said light chain CDR2 is substituted by a different amino acid;
(iii) a light chain CDR3 of SEQ ID NO: 15 or a variant thereof wherein 1, 2, or 3 amino acids of said light chain CDR3 is substituted by a different amino acid.

5. The isolated antibody or fragment thereof according to claim 4, wherein the light chain variable region comprises amino acid sequence SEQ ID NO: 17 or SEQ ID NO: 16, or a variant thereof wherein 1, 2, or 3 amino acids of at least one of the light chain CDR1, CDR2, and/or CDR3, and/or 1, 2, 3, 4, 5, 6, 7, 8, 9, 10, 11, 12, 13, 14, or 15 amino acids of the light chain variable framework region is substituted by a different amino acid.

6. The isolated antibody or fragment thereof according to any one of claims 4 and 5, wherein the light chain variable region comprises amino acid sequence SEQ ID NO: 17.

7. The isolated antibody or fragment thereof according to any one of claims 1 to 6, which inhibits the catalytic activity of MMP9.

8. The isolated antibody according to any one of claims 1 to 7, which is a human monoclonal antibody.

9. An isolated nucleic acid molecule encoding an antibody or antigen binding fragment thereof according to any one of claims 1 to 8.

10. The isolated nucleic acid according to claim 9 comprising one or more of:
(1) a nucleic acid sequence comprising SEQ ID NO: 18, SEQ ID NO: 19, and SEQ ID NO: 20, or a variant thereof having at least 80%, at least 90%, at least 95%, at least 96%, at least 97%, at least 98%, or at least 99% identity with one of said sequences, and/or
(2) a nucleic acid sequence comprising SEQ ID NO: 23, SEQ ID NO: 24, and SEQ ID NO: 25, or a variant thereof having at least 80%, at least 90%, at least 95%, at least 96%, at least 97%, at least 98%, or at least 99% identity with one of said sequences.

11. A recombinant expression vector comprising a nucleic acid molecule according to any one of claims 9 to 10.

12. A host cell comprising a recombinant vector according to claim 11.

13. A process for producing antibodies or fragments thereof according to any one of claims 1 to 8 comprises culturing a host cell transformed with an expression vector comprising a nucleic sequence that encodes said antibodies or fragments thereof under conditions sufficient to promote expression of said antibodies or fragments thereof.

14. A pharmaceutical composition comprising one or more of (i) an antibody specific for MMP9 or antigen-binding fragment thereof according to any one of claims 1 to 8, (ii) a nucleic acid according to any one of claims 9 to 10, (iii) a vector according to claim 11, and/or (iv) a host cell according to claim 12, and at least one pharmaceutically acceptable carrier.

15. The antibody or fragment thereof according to any one of claims 1 to 8 for use in the prevention and/or treatment of a MMP9-related disease selected from the group consisting of inflammatory and autoimmune diseases, cancers or tumors, lung diseases, fibrotic lung diseases, septicemia, muscular dystrophy, allergy, renal fibrosis, scleroderma, dilated cardiomyopathy, Chagas disease, cardiovascular diseases, neuropsychiatric disorders, diabetes, eye diseases, or any other MMP9-related disease or disorder.
